# EUROPEAN PATENT APPLICATION

(11) **EP 2 394 666 A2**
(43) Date of publication of application: **14.12.2011**
(21) Application number: 10708231.5
(22) Date of filing: 04.02.2010
(51) Int. Cl.: A61K 47/48, A61K 9/107, A61K 47/40, A61P 43/00

(54) **PHARMACEUTICAL COMPOSITIONS COMPRISING TGF- BETA 1 INHIBITOR PEPTIDES**

(30) Priority: 05.02.2009 ES 200900319
(71) Applicant: Digna Biotech, S.L., 31008 Pamplona Navarra (ES)
(72) Inventor: IRACHE GARRETA, Juan Manuel, E-31008 Pamplona (Navarra) (ES); MARTÍNEZ GALÁN, Fernando, E-31008 Pamplona (Navarra) (ES)
(74) Representative: ABG Patentes, S.L.
(86) International application number: PCT/ES2010/070062
(87) International publication number: WO 2010/089443

(57) **Abstract**

The present invention relates to a complex comprising a TGF-β1 inhibitor peptide and a cyclodextrin or a derivative thereof. A TGF-β1 inhibitor peptide emulsion comprising cetyl PEG/PPG-10/1 dimethicone is also provided. Processes for the preparation of said complex and said TGF-β1 inhibitor peptide emulsion are also described, as well as its use for the manufacture of a medicament for the treatment of a disease or condition mediated by a TGF-β1.

## Description

### FIELD OF THE INVENTION

The technical field of the invention is pharmaceutical sciences, particularly galenic formulations.

### BACKGROUND OF THE INVENTION

Transforming growth factor-beta (TGF-β) denotes a family of proteins, TGF-β1, TGF-β2, and TGF-β3, which are pleiotropic modulators of cell growth and differentiation, embryonic and bone development, extracellular matrix formation, hematopoiesis, immune and inflammatory responses (Roberts and Sporn Handbook of Experimental Pharmacology (1990) 95: 419-58; Massagué et al. Ann Rev Cell Biol (1990) 6: 597-646). Other members of this superfamily include activin, inhibin, bone morphogenic protein, and Mullerian inhibiting substance. TGF-β initiates an intracellular signaling pathway leading ultimately to the expression of genes that regulate the cell cycle, control proliferative responses, or relate to extracellular matrix proteins that mediate outside-in cell signaling, cell adhesion, migration, and intercellular communication.

TGF-β superfamily members critically regulate many different processes within the cardiovascular and the female reproductive systems. TGF-β superfamily members are abundantly expressed in the bone environment and regulate a number of important bone processes. The contribution of TGF-β signaling to human cancer and connective tissue diseases has been extensively studied. TGF-β superfamily signaling is also critical during embryogenesis from the initial stages of blastula formation, during gastrulation, and throughout the multiple stages of organ development. TGF-β superfamily members are continually being linked to other diseases such as neurological disorders, otosclerosis, psoriases, biliary cirrhosis, and childhood asthma. The range of diseases in which TGF-β superfamily members have a role will likely continue to grow (Gordon and Blobe Biochimica et Biophysica Acta (2008) 1782:197-228).

Due to the ubiquitous role of the TGF-β superfamily in several pathological processes, different antagonist molecules have been devised to neutralize TGF-β1 activity, including small molecules as well as antisense oligonucleotides and antibodies.

WO2000/31135 (Proyecto de Biomedicina Cima, S.L.) relates to peptides antagonists of the binding of TGF-β1 to the type III TGF-β1 receptor or endoglin. WO2007/048857 (Proyecto de Biomedicina Cima, S.L) relates to the use of a peptide inhibiting TGF-β1 selected from: peptide P144 whose sequence corresponds to SEQ ID NO: 1, peptide p 17 whose sequence corresponds to SEQ ID NO: 2, a peptide which has at least 90% homology therewith, or fragments of the above, in the preparation of an immune response modulating agent.

Owing to the clinical development of TGF-β1 inhibitors peptide, galenic formulations are increasingly in need for the administration of these antagonists to the body.

In this respect, US2007/0207965 provides a method for the treatment of skin fibrosis with a peptide that inhibits TGF-β, and suitable compositions for its administration. The method includes in particular the use of peptide P144. For the administration of this peptide, stable emulsions with dimethylsulfoxide as the solubilizing agent are also supplied (the 965 emulsion or E.965). The lipophilic phase comprises dimethicone 350 and a mineral oil. Said emulsion can be loaded with up to 300 µg of peptide per ml, and its storage conditions require temperatures around 5°C.

Drugs of peptidic nature are usually challenging to formulate. In particular, the TGF-β peptide inhibitor P144 is insoluble in water, polyethyleneglycol 400 (PEG400), ethanol, glycerol, polyoxyethylene-polyoxypropylene block copolymer (Pluronic® F68), tetrahydrofuran, hexane, dimethylformamide, dichloromethane, polyvinyl pyrrolidone (Plasdone®), propylene glycol, Gantrez^{®} S97, sodium deoxycholate, to name a few solvents.

Cyclodextrins are known in the art as a plausible means for solubilizing therapeutic compounds. Cyclodextrins are cyclic oligosaccharides built up from six or more α-D-glucopyranoside units forming a rigid conical molecular structure with hollow interiors of specific volumes. They are able to form host-guest complexes with certain hydrophobic molecules, modifying the physical and chemical properties of the guest molecule, mostly in terms of water solubility.

In this respect, WO2008/013928 (Biogen IDEC MA Inc., The Trustees of the University of Pennsylvania) relates to methods for cancer treatment, comprising administering to a subject in need of the treatment a combination therapy which comprises (a) administration of a TGF-β inhibitor and (b) a vector comprising an isolated polynucleotide which encodes an interferon polypeptide. The TGF-β inhibitor includes soluble human TGFRIII polypeptides. Solubilizing agents such as cyclodextrins, or other solubilizing agents well-known to those familiar with the art, can be utilized as pharmaceutical excipients for delivery of the therapeutic compounds. Yet, this patent publication does not provide actual formulations comprising a TGF-β inhibitor and a cyclodextrin.

WO2003/048323 (Bristol-Myers Squibb Company) relates to polypeptides in the diagnosis, treatment, and prevention of rheumatoid arthritis and related disease states, wherein said polypeptide associated with rheumatoid arthritis has an amino acid sequence which is at least 95% identical to the amino acid sequence SEQ ID NO: 48. SEQ ID NO: 48 corresponds to Human Transforming Growth Factor-Beta Type III Receptor amino acid sequence (Accession No. Q03167). The compound(s) of the invention may be formulated with fast dissolving diluents such as mannitol, lactose, sucrose and/ or cyclodextrins. Similarly, the effective activity of cyclodextrins for solubilizing TGFBRIII polypeptides is not demonstrated.

The formation of cyclodextrin complexes is dependent on geometric and structural affinities between the cyclodextrin and the guest molecules, as well as the volume of the cyclodextrin inner cavity. Thus, not all molecules are able to form complexes with cyclodextrins, or with particular types thereof. As such, it remains to be demonstrated whether TGF-β1 inhibitor peptides can complex with cyclodextrins, and which types of cyclodextrins are suitable for forming these molecular encapsulations.

Not being sufficient to solubilize TGF-β1 inhibitor peptides, the formulators are still required to fulfill the ultimate goal of providing final formulations that can effectively deliver the active compounds to the body. In this respect, lipid-based formulations like emulsions appear as a promising vehicle system for delivering poorly soluble drugs. Emulsions are an intimate mixture of two incompletely miscible liquids, as oil and water, in which one of the liquids in the form of fine droplets is dispersed in the other usually with the aid of an emulsifier or surfactant.

Selection of a suitable surfactant for preparing sufficiently stable emulsions for a particular application is not a predictable or routine exercise. Furthermore, in the particular case of preparing increased dosages of TGF-β1 inhibitor peptides, other parameters such as the reduction of drug crystallization and precipitation need to be considered.

In an exhaustive effort to improve the physicochemical properties of TGF-β1 inhibitor peptides, particularly in terms of solubility, and set up with the task of improving the 965 emulsion (E.965) formulation, the inventors of the present invention have successfully obtained cyclodextrin complexes with the TGF-β1 inhibitor peptides of interest, and concocted an emulsion comprising these cyclodextrin complexes and the TGF-β1 inhibitor peptides and the surfactant cetyl PEG/PPG-10/1 dimethicone (Abil® EM90), which meets one or more of the objectives mentioned below.

It is an object of the present invention, the provision of a formulation that improves the water solubility of TGF-β1 inhibitor peptides.

It is an object of the present invention, the provision of a formulation that improves the water solubility of TGF-β1 inhibitor peptides, which avoids the presence or diminishes the quantity of organic solvents.

It is an object of the present invention, the provision of a safe TGF-β1 inhibitor peptide formulation that employs pharmaceutically acceptable excipients.

It is an object of the present invention, the provision of TGF-β1 inhibitor peptides emulsions that are sufficiently stable, with suitable shelf-life or chemical stability.

It is an obj ect of the present invention, the provision of crystal free or crystal negligible TGF-β1 inhibitor peptides emulsions.

It is an object of the present invention, the provision of TGF-β1 inhibitor peptides emulsions that are sufficiently homogeneous.

It is an object of the present invention, the provision of TGF-β1 inhibitor peptides emulsions with an increased drug load per composition.

It is an object of the present invention, the provision of TGF-β1 inhibitor peptides emulsions with a fine particle grade (of the inner particle).

It is an object of the present invention, the provision of TGF-β1 inhibitor peptides emulsions that can be prepared at mild conditions, for example, employing less heating. It is an object of the present invention, the provision of TGF-β1 inhibitor peptides emulsions that are suitable for industrial upscale production.

It is an object of the present invention, the provision of a formulation that allows the topical local action of TGF-β1 inhibitor peptides without being absorbed or being minimally absorbed.

It is an object of the present invention, the provision of a formulation of TGF-β1 inhibitor peptides that admits different ways of administration to the body, e.g., via oral, parenteral, or topical routes.

It is an object of the present invention, the provision of TGF-β1 inhibitor peptides emulsions easily spreadable.

It is an object of the present invention, the provision of TGF-β1 inhibitor peptides emulsions of a pleasant appearance without being greasy.

In one aspect the present invention relates to a complex comprising a TGF-β1 inhibitor peptide and a cyclodextrin or a derivative thereof, wherein
- the TGF-β1 inhibitor peptide is selected from a peptide having at least 70% sequence identity to one amino acid sequences of SEQ ID NO: 1-23,salts, funtionally derivatives, variants, analogues and fragments thereof capable of inhibiting TGF-β1; and
- the cyclodextrin or a derivative thereof is selected from a β-cyclodextrin and a γ-cyclodextrin.

In another aspect, the present invention relates to a pharmaceutical formulation comprising said complex, in which the TGF-β1 inhibitor peptide is present in a therapeutically effective amount and cyclodextrin is a cyclodextrin pharmaceutically acceptable. In a particular embodiment, said pharmaceutical formulation comprises cetyl PEG/PPG-10/1 dimethicone.

In another aspect, the present invention relates to a TGF-β1 inhibitor peptide emulsion **characterized in that** said emulsion comprises cetyl PEG/PPG-10/1 dimethicone.

In another aspect, the present invention relates to a pharmaceutical formulation comprising said TGF-β1 inhibitor peptide emulsion, in which said TGF-β1 inhibitor peptide is present in a therapeutically effective amount. In a particular embodiment of said emulsion, said TGF-β1 inhibitor peptide forms a complex with a cyclodextrin or a derivative thereof, and said cyclodextrin is a pharmaceutically acceptable cyclodextrin.

In another aspect the present invention relates to a procedure for the preparation of said complex comprising a TGF-β1 inhibitor peptide and a cyclodextrin or a derivative thereof comprising said procedure the mixture of the TGF-β1 inhibitor peptide with an aqueous solution comprising the cyclodextrin or a derivative thereof. The product obtained by this procedure constitutes an additional aspect of this invention.

In another aspect the present invention relates to a procedure for the preparation of said TGF-β1 inhibitor peptide emulsion comprising cetyl PEG/PPG-10/1 dimethicone. , said procedure comprising:
a) the preparation of (i) a complex by mixing the TGF-β1 inhibitor peptide with an aqueous solution comprising the cyclodextrin or a derivative thereof; and (ii) a lipophilic phase comprising cetyl PEG/PPG-10/1 dimethicone; and
b) the admixture of said complex onto said lipohilic phase.

The product obtained by this procedure constitutes an additional aspect of this invention.

In another aspect the present invention relates to the use of said complex, said TGF-β1 inhibitor peptide emulsion, or either said pharmaceutical formulations , for the manufacture of a medicament for the treatment of a disease or condition mediated by TGF-β1. In another aspect, the present invention relates to a method of treating a disease or condition mediated by TGF-β1 in a mammal, said method comprising the administration of an effective amount of said complex, or said TGF-β1 inhibitor peptide emulsion, or either said pharmaceutical formulations.

### BRIEF DESCRIPTION OF THE FIGURES

Figure 1: Microscopy images of siliconic emulsion with 100 µg/g, batch A-2, on the left with visible light and on the right with UV light and subsequently processed in order to facilitate the view in black and white (conversion to color format CMYK; selection of channel or black component; grey inversion, which is represented in the image).
Figure 2: Microscopy images of blank siliconic emulsion, batch A-3, on the left with visible light and on the right with UV light and processed as previously mentioned.
Figure 3: Microscopy images of siliconic emulsion, batch A-10, on the left with visible light and on the right with UV light and processed as previously mentioned (concentration of P144: 1,000 µg/g).
Figure 4: Microscopy images of siliconic emulsion, batch A-16, on the left with visible light and on the right with UV light and processed as previously mentioned (concentration of P144: 1,000 µg/g).
Figure 5: Microscopy images of siliconic emulsion, batch A-18, on the left with visible light and on the right with UV light and processed as previously mentioned (concentration of P144: 700 µg/g). Crystals of P144 are indicated by arrows.
Figura 6: Microscopy images of siliconic emulsion with a concentration of P144 of 1,100 µg/g, batch A-28, a la izquierda con luz visible y a la derecha con luz ultravioleta. Crystals of P144 are indicated by arrows.
Figure 7: Microscopy images of siliconic emulsion with a concentration of P144 of 800 µg/g, batch A-30, on the left with visible light and on the right with UV light and processed.
Figure 8: Photograph of P144 emulsion called (ESD600).

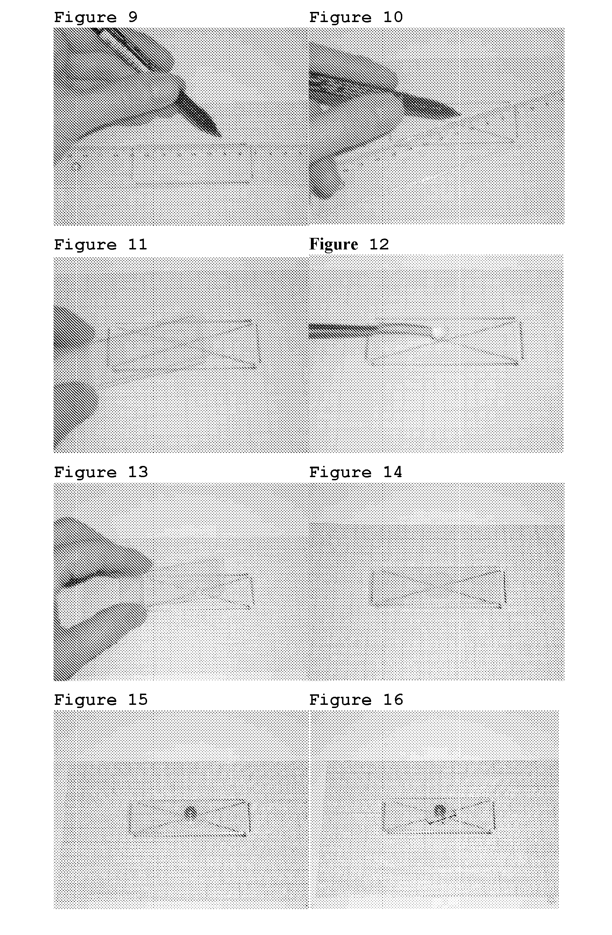

Figures 9-16: Measurement of the expansion capacity of P144 emulsion (ESD600) [Example 6, paragraph 2.2.]; proceeded to trace the sides of a slide on millimetered paper (Figure 9) and to trace the diagonals (Figure 10); next, the lower slide is positioned coinciding with the previous tracing (Figure 11) and the sample of P144 emulsion ESD600 is placed at the point of intersection of the diagonals (Figure 12). Then, the upper slide is positioned on the lower slide containing the sample of the emulsion (Figure 13) and the diameter of the circle, formed by the emulsion as a result of the weight of the overlying slide, is measured (Figure 14); next a known weight is placed on the sample, in the intersection diagonals, (Figure 15) and the diameters following the traced diagonals are measured (Figure 16).
Figure 17: Representation of the surface increment of the P144 emulsion (ESD600) as a function of the applied weight.
Figure 18: Determination of the external phase of P144 emulsion (ESD600) using the dilution method.
Figure 19: Determination of the external phase of P144 emulsion (ESD600) using the dilution method.
Figure 20: Determination of the external phase of P144 emulsion (ESD600) using the dye method.
Figure 21: Amount of P144 in the receptor of Franz cell. ES-0: control Siliconic Emulsion ESD600_{[0]}; ES-100: Siliconic Emulsion ESD600 with 100 µg P144/g (ESD600_{[100]}). Data represents meant ± standard deviation (SD) (n = 4 for ES-0 and n = 8 for ES-100).
Figure 22: Comparison of the quantification of the amount of P144 in the different areas of the pig ear skin. Each column represents the mean value of the concentration of P144 retained in the skin section, expressed as milligrams of P144/cm³ of skin + SD (n = 8). CD-100: Emulsion E.965 with 100 µg P144/g (E.965_{[100]}); ES-100: Siliconic Emulsion ESD600_{[100]} with 100 µg P144/g.
Figure 23: Quantification of the amount of P144 in the different areas of the pig ear skin. Each column represents the mean value of the concentration of P 144 retained in the skin section, expressed as milligrams of P144/cm³ of skin + SD (n = 8). CD-100: Emulsion E.965 with 100 µg P144/g (E.965_{[100]}); ES-100: Siliconic Emulsion ESD600_{[100]} with 100 µg P144/g.
Figure 24: Representation of the theoretical amount of P 144 in the different performed cuts. A 100% would be achieved when all drug had passed from the semisolid preparation to the skin (pig skin: 176 mm² x 1 mm in depth). CD-100: Emulsion E.965 with 100 µg P144/g (E.965_{[100]}); ES-100: Siliconic emulsion ESD600_{[100]} with 100 µg P144/g.

### DETAILED DESCRIPTION OF THE INVENTION

In one aspect, the present invention relates to a complex henceforth "complex of the invention" comprising a TGF-β1 inhibitor peptide and a cyclodextrin or a derivative thereof, wherein
- the TGF-β1 inhibitor peptide is selected from a peptide having at least 70% sequence identity to one amino acid sequences of SEQ ID NO: 1-23 its salts, funtionally derivatives, variants, analogues and fragments thereof capable of inhibiting TGF-β1; and
- the cyclodextrin or a derivative thereof is selected from a β-cyclodextrin and a γ-cyclodextrin.

The term "complex" refers to a structure in which one or more cyclodextrin molecules (the "host") form a cavity within which one or more molecules of a second compound (the "guest") are located. As used herein, the second compound refers to a TGF-β1 inhibitor peptide. The terms complex and molecular encapsulation may be used interchangeably.

The term "TGF-β1 inhibitor peptide" refers to a molecule having the ability to inhibit a biological function of TGF-β1 by interacting with the active form of TGF-β1, particularly the TGF-beta isoform 1 with accession number AAL27646 or NP 000651. While the inhibitors herein are characterized by their ability to interact with TGF-beta 1, they might additionally interact with the other mammal isoforms (TGF-β2 and β3). This activity inhibits TGF-beta interaction with the corresponding superfamily receptors, like type I receptors such as activin like kinases ALK1, ALK2, ALK5; type II receptors such as type II TGF-β receptor (TβRII); co-receptors such as endoglin and crypto. Additionally, the inhibitors may as well interact with type I receptors like ALK3, ALK4, ALK6, ALK7; type II receptors like ActRII, ActRIIb, BMPRII, MISRII, and TβRII; co-receptors like RGMa, RGMb, and hemojuvelin; pseudo-receptors like BAMBI; signaling components such as chordin, follistatin, lefty 1, noggin, sclerostin; and other members in the TGF-β signal transduction pathway or members shared by the TGF-β signal transduction pathway and another pathway.

These molecules are of peptidic nature, meaning that they comprise α-amino acids linked by an amide bond, i.e. the peptidic bond. The term "peptide" is not to be limited to short amino acid chains; it can include chains of more than 50 amino acids in length. As such, the term peptide as used herein encompasses as well polypeptides and proteins.

In the present invention, the TGF-β1 inhibitor peptide is preferably selected from a peptide having at least 70%, advantageously at least 80% or preferably at least 90%, sequence identity to one amino acid sequences of SEQ ID NO: 1-23 as depicted in the Table 1 hereunder.

**Table 1**

| Internal reference | SEQ ID NO: | CAS Registry number | Sequence |
|---|---|---|---|
| P11(WO0031135,SEQ ID NO:1) | 1 | 271791-38-9 | HANFCLGPCPYIWSL |
| P12 | 2 | 271791-39-0 | FCLGPCPYIWSLDT |
| P54 | 3 | 271791-82-3 | TSLDATMIWTMM |
| P106 | 4 | 272105-04-1 | SNPYSAFQVDIIVDI |
| P142 | 5 | 272105-40-5 | TSLMIWTMM |
| P144 | 6 | 272105-42-7 | TSLDASIIWAMMQN |
| P145 | 7 | 272105-43-8 | SNPYSAFQVDITID |
| P150 | 8 | 272105-47-2 | EAVLILQGPPYVSWL |
| P152 | 9 | 272105-49-4 | LDSLSFQLGLYLSPH |
| P29 | 10 | 271791-56-1 | HEPKGYHANFCLGPCPYIWSLDT |
| P11(WO2005019244, SEQ ID NO:11) | 11 | 846546-30-3 | WHKYFLRRPLSVRTR |
| P3 | 12 | 846546-23-4 | RFFTRFPWHYHASRL |
| P13 | 13 | 846546-32-5 | RKWFLQHRRMPVSVL |
| P14 | 14 | 846546-33-6 | SGRRHLHRHHIFSLP |
| P4 | 15 | 846546-24-5 | RLAHSHRHRSHVALT |
| P6 | 16 | 846546-26-7 | PPYHRFWRGHRHAVQ |
| P17 | 17 | 846546-36-9 | KRIWFIPRSSWYERA |
| P18 | 18 | 846546-37-0 | MPLSRYWWLFSHRPR |
| P17(1-14) | 19 | 846546-51-8 | KRIWFIPRSSWYER |
| P17(1-12) | 20 | 846546-52-9 | KRIWFIPRSSWY |
| P17(1-11) | 21 | 934621-99-5 | KRIWFIPRSSW |
| P17(1-11)am | 22 | 934622-00-1 | KRIWFIPRSSW; Trp-11; C-terminal amide |
| AcP17(1-11)am | 23 | 934622-01-2 | KRIWFIPRSSW; Lys-1;- N-acetyl Trp-11; C-terminal amide |

TGF-β1 inhibitor peptides may be obtained from a variety of cell sources that synthesize these peptides including, for example, cells transfected with recombinant DNA molecules capable of directing the synthesis or secretion of the peptides. Alternatively, TGF-β1 inhibitor peptides may be synthesized by chemical synthetic methods, including but not limited to, solid phase peptide synthesis. The peptides are as well commercially available, for instance P144 is supplied by Sigma-Genosys, Ltd. (Cambridge, UK).

As known in the art, "sequence identity" between two (poly)peptides or proteins is determined by comparing the amino acid sequence of one (poly)peptide to the sequence of a second (poly)peptide. As discussed herein, whether a particular (poly)peptide is at least about 70%, 75%, 80%, 85%, 90% or 95% identical to another (poly)peptide can be determined using methods and computer programs or software known in the art such as, but not limited to, the BESTFIT program (Wisconsin Sequence Analysis Package, Version 8 for Unix, Genetics Computer Group) or the GCG package (GAP version 8, Genetics Computer Group, USA). BESTFIT uses the local homology algorithm of Smith and Waterman (Advances in Applied Mathematics 2:482-489 (1981)), to find the best segment of homology between two sequences. The GCG package uses standard penalties for proteins: GAP weight 3.00, length weight 0.100, and the Matrix described in Gribskov and Brugess, Nucl. Acids Res. (1986) 14(16), 6745-6763.

In a particular embodiment, the TGF-β1 inhibitor peptide is a peptide selected from the group of peptides whose sequence is shown in SEQ ID NO: 1-23, or a salt, functional derivative, variant, analog, or fragment thereof with capacity to inhibit TGF-β1. Therefore, in a particular embodiment, the TGF-β1 inhibitor peptide is a peptide whose amino acid sequence is shown in SEQ ID NO:1, or in SEQ ID NO:2, or in SEQ ID NO:3 or in SEQ ID NO:4, or in SEQ ID NO:5, or in SEQ ID NO:6, or in SEQ ID NO:7, or in SEQ ID NO:8, or in SEQ ID NO:9, or in SEQ ID NO:10, or in SEQ ID NO:11, or in SEQ ID NO:12, or in SEQ ID NO:13, or in SEQ ID NO:14, or in SEQ ID NO:15, or in SEQ ID NO:16, or in SEQ ID NO:17, or in SEQ ID NO:18, or in SEQ ID NO:19, or in SEQ ID NO:20, or in SEQ ID NO:21, or in SEQ ID NO:22, or in SEQ ID NO:23, or a salt, a functional derivative, a variant, an analog, or a fragment thereof with capacity to inhibit TGF-β1.

In another particular embodiment, the TGF-β1 inhibitor peptide is selected from a peptide having at least 70%, advantageously at least 80%, preferably at least 90%, sequence identity to the amino acid sequences of SEQ ID NO: 1-23, or a salt, a functional derivative, a variant, an analog, or a fragment thereof with capacity to inhibit TGF-β1.

Therefore, in a particular embodiment, the TGF-β1 inhibitor peptide is a peptide whose amino acid sequence has at least 70%, advantageously at least 80%, preferably at least 90%, sequence identity to the amino acid sequences shown in SEQ ID NO: 1, or in SEQ ID NO: 2, or in SEQ ID NO: 3 or in SEQ ID NO: 4, or in SEQ ID NO: 5, or in SEQ ID NO: 6, or in SEQ ID NO: 7, or in SEQ ID NO: 8, or in SEQ ID NO: 9, or in SEQ NO: 10, or in SEQ ID NO:11, or in SEQ ID NO:12, or in SEQ ID NO:13, or in SEQ ID NO:14, or in SEQ ID NO:15, or in SEQ ID NO:16, or in SEQ ID NO:17, or in SEQ ID NO:18, or in SEQ ID NO:19, or in SEQ ID NO:20, or in SEQ ID NO:21, or in SEQ ID NO:22, or in SEQ ID NO:23, or a salt, a functional derivative, a variant, an analog, or a fragment thereof with capacity to inhibit TGF-β1.

The term "salts" herein refers to both salts of carboxyl groups and to acid addition salts of amino groups of the TGF-β1 inhibitor peptides. Salts of a carboxyl group may be formed by means known in the art and include inorganic salts, for example, sodium, calcium, ammonium, ferric, or zinc salts, and the like, and salts with organic bases as those formed, for example, with amines, such as triethanolamine, arginine, lysine, piperidine, procaine, and the like. Acid addition salts include, for example, salts with mineral acids, such as, for example, hydrochloric acid or sulfuric acid, and salts with organic acids, such as, for example, acetic acid or oxalic acid. Of course, any such salt must retain the biological activity of the TGF-β1 inhibitor peptide. For therapeutic use, salts of TGF-β1 inhibitor peptides are those wherein the counter-ion is pharmaceutically acceptable. Non-pharmaceutically acceptable salts are also encompassed in the ambit of the present invention since they can be used in the production of pharmaceutically acceptable end products.

"Functional derivatives" as used herein covers derivatives which may be prepared from the functional groups which occur as side chains on the residues or the N- or C-terminal groups, by means known in the art, and are included in the invention as long as they remain pharmaceutically acceptable, i.e., they do not destroy the biological activity of the peptides as described above, i.e., the ability to bind the corresponding receptor and initiate receptor signaling, and do not confer toxic properties on compositions containing it. Derivatives may have chemical moieties, such as carbohydrate or phosphate residues, provided such a derivative retains the biological activity of the peptide and remains pharmaceutically acceptable.

For example, derivatives may include aliphatic esters of the carboxyl groups, amides of the carboxyl groups by reaction with ammonia or with primary or secondary amines, N-acyl derivatives or free amino groups of the amino acid residues formed with acyl moieties (e.g., alkanoyl or carbocyclic aroyl groups), or O-acyl derivatives of free hydroxyl group (e.g., that of seryl or threonyl residues) formed with acyl moieties. Such derivatives may also include for example, polyethylene glycol side-chains, which may mask antigenic sites and extend the residence of the molecule in body fluids.

A "variant" according to the present invention refers to a molecule, which is substantially similar to either the entire peptides defined above or a fragment thereof. Variant peptides may be conveniently prepared by direct chemical synthesis of the variant peptide, using methods well known in the art. Of course, such variant would have similar receptor binding and signal initiating activity as the corresponding TGF-β1 inhibitor peptides.

Variations in the peptide primary structure, as well as variations in higher levels of structural organization, e.g. variation in the type of covalent bonds linking the amino acid residues or addition of groups to the terminal residues of the peptide, are within the scope of the invention. Further, the peptide molecules may include conservative or non-conservative alterations in the amino acid sequence that result in silent changes that preserve the functionality of the molecule including, for example, deletions, additions, and substitutions. Such altered molecules may be desirable where they provide certain advantages in their use. As used herein, conservative substitutions would involve the substitution of one or more amino acids within the sequence of the corresponding peptide with another amino acid having similar polarity and hydrophobicity/hydrophilicity characteristics resulting in a functionally equivalent molecule. Such conservative substitutions include but are not limited to substitutions within the following groups of amino acids: glycine, alanine; valine, isoleucine, leucine; aspartic acid, glutamic acid; asparagine, glutamine; serine, threonine; lysine, arginine; phenylalanine, tyrosine; and methionine, norleucine.

Amino acid sequence variants of the peptide defined above can be prepared by mutations in the DNAs, which encode the synthesized derivatives. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure.

At the genetic level, these variants ordinarily are prepared by site-directed mutagenesis of nucleotides in the DNA encoding the peptide molecule, thereby producing DNA encoding the variant, and thereafter expressing the DNA in recombinant cell culture. The variants typically exhibit the same qualitative biological activity as the non-variant peptide.

An "analog" of the peptides defined above, according to the present invention, refers to a non-natural molecule, which is substantially similar to either, the entire molecules or to an active fragment thereof. Such analog would exhibit the same activity as the corresponding TGF-β1 inhibitor peptides.

A "fragment" according to the present invention refers to any subset of the molecules, that is, a shorter peptide, which retains the desired biological activity, for example the capacity of inhibit TGF-β1. Fragments may readily be prepared by removing amino acids from either end of the molecule and testing the resultant for its properties as a receptor antagonist. Proteases for removing one amino acid at a time from either the N-terminal or the C-terminal of a polypeptide are known in the art.

The desired biological activity of TGF-β1 inhibitor peptide (e.g. the capacity of inhibit TGF-β1) may be determined by any suitable conventional bioassay for measuring TGF-β lactivity, for example by the assays described by Meager Journal of Immunological Methods (1991) 141: 1-14. Amongst these methods, the Mv-1-Lu cell growth inhibition assay is particularly suitable. A description of the Mv-1-Lu cell assay is also provided in WO2005/019244. Preferably, the TGF-β1 inhibitor peptide of the present invention has an inhibitory activity of 20% or higher in the Mv-1-Lu cell growth inhibition assay. More preferably the TGF-β1 inhibitor peptide of the present invention has an inhibitory activity of at least 50% in the Mv-1-Lu cell growth inhibition assay.

The term "cyclodextrin" as used herein includes any of the known cyclodextrins such as unsubstituted cyclodextrins containing from six to twelve glucose units, especially, α-cyclodextrin, β-cyclodextrin, γ-cyclodextrin, their derivatives, and mixtures thereof. The α-cyclodextrin consists of six glucose units, the β-cyclodextrin consists of seven glucose units, and the γ-cyclodextrin consists of eight glucose units, arranged in donut-shaped rings.

The term "cyclodextrin derivative" as used herein, includes any cyclodextrin with, at least, one terminal hydroxyl group modified. Chemical modification of cyclodextrins can alter their physicochemical properties, improving the solubility, stability and controlling the chemical activity of molecules with which they are connected (guest molecules). Incorporation has been described by reaction of the hydroxyl groups (OH) of cyclodextrins, alkyl groups, aryl carboxialquil, cyanoalkyl, hydroxyalkyl, sulfo-alkyl, amino, azido, heterocyclyl, acetyl, benzoyl, succinyl, and other groups containing phosphorus, sulfur... [Robyt (1998) "Essentials of carbohydrate chemistry", Ed Charles R. Cantor, Springer Advanced Text in Chemistry].

Preferable cyclodextrins to form complexes with the TGFβ1 peptide inhibitors are β-cyclodextrins and γ-cyclodextrins. More preferable are the hydrophilic derivatives of β-cyclodextrins and γ-cyclodextrins, such as 2-hydroxypropyl-β-cyclodextrin, 2-hydroxyethyl-γ-cyclodextrin, 2-hydroxypropyl-γ-cyclodextrin, or sulfated β-cyclodextrin and sulfated β-γ-cyclodextrin. Ilustrative exemples of derivatives of β-cyclodextrins include, without being limited to, mono-or poly- (C₁-C₆) alkylated β-cyclodextrin such as dimethyl-β-cyclodextrin or heptakis (2,6-di-0-methyl)-β-cyclodextrin (DIMEB), trimethyl-β-cyclodextrin or heptakis (2,3,6-tri-0-methyl)-β-cyclodextrin (TRIMEB), random methylated β-cyclodextrin (RM-β-CD); mono-or poly-(C₁-C₆) hydroxyalkylated β-cyclodextrin such as hydroxyethyl-β-cyclodextrin, 2-hydroxypropyl-β-cyclodextrin (HP-β-CD), hydroxybutyl-β-cyclodextrin; carboxy (C₁-C₆) alkylated β-cyclodextrins such as carboxymethyl-β-cyclodextrin, carboxyethyl-β-cyclodextrin; (C₁-C₆) alkylcarbonylated β-cyclodextrins such as acetyl-β-cyclodextrin; mono-, tetra-, or hepta-substituted β-cyclodextrin; sulfoalkyl ether cyclodextrins (SAE-CD) such as sulfobutylether cyclodextrin (SBECD); maltosyl-β-cyclodextrin; (2-carboxymethoxy)propyl-β-cyclodextrin, etc

The cyclodextrins and derivatives thereof for use in the present invention are commercially available for instance from Sigma-Aldrich. They may also be synthesized according to methods known by the skilled in the art.

The amount of TGF-β1 inhibitor peptide present in the complex of the invention can vary within a wide range, nevertheless in one particular embodiment of the present invention, the TGF-β1 inhibitor peptide is present in the complex of the invention in a weight ratio of about 0.002:1 to about 0.024:1 relative to the cyclodextrin (0.002-0.024:1). In another particular embodiment, the TGF-β1 inhibitor peptide is present in the complex of the invention in a weight ratio of about 0.004:1 to about 0.018:1 relative to the cyclodextrin (0.004-0.018:1). In another particular embodiment, the TGF-β1 inhibitor peptide is present in the complex of the invention in a weight ratio of about 0.004:1 to about 0.024:1 relative to the cyclodextrin (0.004-0.024:1). In another particular embodiment, the TGF-β1 inhibitor peptide is present in the complex of the invention in a weight ratio of about 0.002:1 to about 0.018:1 relative to the cyclodextrin (0.002-0.018:1). The range of workable ratios between the TGF-β1 inhibitor peptide and the cyclodextrin may depend on other excipients present in the emulsion formulations of any one of the embodiments described herein. Preferably the complex comprises the TGF-β1 inhibitor peptide of SEQ ID NO: 6 and HP-β-CD. More preferably the complex of the invention comprises the TGF-β1 inhibitor peptide of SEQ ID NO: 6 and HP-β-CD in a weight ratio of about 0.004:1 to about 0.018:1 relative to the cyclodextrin (0.004-0.018:1). Even more preferably, the complex of the invention comprises the TGF-β1 inhibitor peptide of SEQ ID NO: 6 and HP-β-CD in a weight ratio of about 0.018:1.

In another aspect, the present invention relates to a pharmaceutical formulation henceforth " pharmaceutical composition [1] of the invention" comprising a complex of the invention, wherein the TGF-β1 inhibitor peptide is present in a therapeutically effective amount, and the cyclodextrin is a pharmaceutically acceptable cyclodextrin.

A "therapeutically effective amount" in this context is an amount sufficient to prophylactically act against, stabilize, or treat a disease or condition mediated by TGF-β1 in subjects suffering from such disease or condition.

The therapeutically effective amount of TGF-β1 inhibitor peptide may be comprise in the range of from 0.01 mg to 50 g per day, from 0.02 mg to 40 g per day, from 0.05 mg to 30 g per day, from 0.1 mg to 20 g per day, from 0.2 mg to 10 g per day, from 0.5 mg to 5 g per day, from 1 mg to 3 g per day, from 2 mg to 2 g per day, from 5 mg to 1,5 g per day, from 10 mg to 1 g per day, from 10 mg to 500 mg per day.

The term "pharmaceutically acceptable" applied to a cyclodextrin in the context of the present invention means having no persistent detrimental effect on the health of the subject being treated. The pharmaceutical acceptability of a cyclodextrin depends, among other factors, on the particular cyclodextrin compound in question, on its concentration in the administered composition, and on the route of administration. For example, use of β-cyclodextrin as an excipient in intravenous or parenteral compositions is limited by hemolytic and nephrotoxic effects, but is generally non-toxic when administered orally.

In a particular embodiment, the pharmaceutical formulation [1] of the invention comprising a complex according of the invention further comprises cetyl PEG/PPG-10/1 dimethicone.

In another aspect the present invention relates to a TGF-β1 inhibitor peptide emulsion henceforth "emulsion of the invention " **characterized in that** said emulsion comprises cetyl PEG/PPG-10/1 dimethicone.

Cetyl PEG/PPG-10/1 dimethicone, ABIL® EM 90, is a non-ionic emulsifier or surfactant based on silicone with an approximate HLB (hydrophilic-lipophilic balance) value of 5. Cetyl PEG/PPG-10/1 dimethicone is commercially available from Degussa.

As such the emulsion of the invention is a water-in-oil (w/o) emulsion that comprises substantially uniform and spherical droplets -the hydrophilic phase- dispersed in a continuous medium -the lipophilic phase. The hydrophilic phase comprises the TGF-β1 inhibitor peptide complexed with a β- or with a γ-cyclodextrin. The lipophilic phase comprises cetyl PEG/PPG-10/1 dimethicone amongst other excipients.

The emulsion of the invention is generally characterized by its thermodynamic stability, and by an average particle size comprised in the micron range, i.e. a diameter between about 0.5 and 20 µm, preferably a diameter comprised between about 0.5 µm and 10 µm, more preferably between 1 and 10 µm. The average particle size is dependant, amongst other factors, on the mixing speed with the aqueous media.

The lipophilic phase of the emulsion of the invention may comprise other surfactants in addition to cetyl PEG/PPG-10/1 dimethicone provided that the surfactant system utilized possesses an overall HLB value comprised between 0 and 8 based on the HLB system. Nevertheless, surfactant compositions comprising PEG/PPG-10/1 dimethicone and one or more additional surfactants with any HLB value and still capable of fanning w/o emulsions are also suitable for the emulsions of the invention. The surfactant composition may therefore include one or more surfactants having a HLB higher than 8, or more hydrophilic in nature, as long as the final surfactant combination with PEG/PPG-10/1 dimethicone is capable of fanning a w/o emulsion; or the overall HLB of the surfactant system is at least smaller than 8. To calculate the final HLB value of the surfactant composition the method by Griffin may be used that further allows the calculation of the relative quantities of the surfactants necessary to produce physically stable formulations for particular water/oil combinations.

In a particular embodiment of the present invention relates to a TGF-β1 inhibitor peptide emulsion of the invention, wherein the TGF-β1 inhibitor peptide is selected from a peptide having at least 70%, advantageously at least 80%, or preferably at least 90% sequence identity to the amino acid sequences of SEQ ID NO: 1-23. Ilustrative examples of emulsions of the invention include those wherein the TGF-β1 inhibitor peptide forms a complex with a cyclodextrin or a derivative thereof.

Particular embodiments relate to emulsions of the invention, wherein the TGF-β1 inhibitor peptide is present in a weight ratio of about 0.002:1 to about 0.024:1 relative to the cyclodextrin (0.002-0.024:1), or in a weight ratio of about 0.004:1 to about 0.024:1 (0.004-0.024:1), or in a weight ratio of about 0.002:1 to about 0.018:1 (0.002-0.018:1), or in a weight ratio of about 0.004:1 to about 0.018:1 (0.004-0.018:1). The range of workable ratios between the TGF-β1 inhibitor peptide and the cyclodextrin may depend on other excipients present in the emulsion formulations e of the invention.

As such, in another aspect, the present invention relates to a pharmaceutical formulation henceforth "pharmaceutical composition [2] of the invention" comprising a emulsion of the invention wherein the TGF-β1 inhibitor peptide is present in a therapeutically effective amount, and the cyclodextrin is a pharmaceutically acceptable cyclodextrin.

In another aspect the present invention relates to a procedure for the preparation of a complex of the invention, said procedure comprising the mixture of the TGF-β1 inhibitor peptide with an aqueous solution comprising the cyclodextrin or a derivative thereof. The product (complex of the invention) obtained by said procedure constitute an additional aspect of the present invention.

In another aspect the present invention relates to a procedure for the preparation of an emulsion of the invention henceforth "procedure for the preparation of an emulsion of the invention", said procedure comprising:
a) the preparation of : (i) a complex by mixing a TGF-β1 inhibitor peptide with an aqueous solution comprising the cyclodextrin or a derivative thereof; and (ii) a lipophilic phase comprising cetyl PEG/PPG-10/1 dimethicone; and
b) the admixture of the complex onto the lipohilic phase.

In one particular embodiment, in the procedure for the preparation of the emulsion, prior to step b), each of the complex and the lipophilic phase are heated at a temperature comprised between 15°C and 70°C, preferably at a temperature between 20°C and 50°C, more preferably at temperature between 25°C and 35°C.

The product (emulsion of the invention) obtained by said procedure for the preparation of the emulsion of the invention, constitute an additional aspect of the present invention.

The various emulsions of the invention are generally prepared by weighing separately the components of each emulsion phase (aqueous and oily phases) in stainless steel vessels of suitable capacity. Then, each phase is heated on a bath suitably with thermostatic control at a temperature comprised between 15°C and 70°C, preferably at a temperature between 20°C and 50°C, more preferably at temperature between 25°C and 35°C. The vessel comprising the aqueous phase may be covered to prevent losses by evaporation. The oily phase is heated until obtaining a homogeneous melt. Preferably, both phases exhibit a clear, homogeneous appearance at temperatures between 25°C and 70°C. Subsequently, the oily phase is stirred, and the aqueous phase is poured onto the oily phase a little at a time. The emulsion is kept at stirring for 30-40 minutes. At the end of the process, the emulsion is at room temperature. The final stage is maturation of the emulsion. This stage is of variable duration, generally between 48 and 72 hours, i.e. a sufficient time for verifying its stability.

A variety of excipients usually utilized in the pharmaceutical arts can be added to the emulsions of the invention. These pharmaceutically acceptable excipients may be preserving agents, emollients, antifoaming agents, antioxidants, buffers, pigments, coloring agents, sweetening agents, flavoring agents, coating agents, granulating agents, disintegrants, glidants, lubricants, conventional matrix materials, complexing agents, absorbents, fillers. They may be used for customary purposes and in typical amounts without adversely affecting the properties of the compositions. The dosage forms of the products provided by the present invention may also contain other therapeutically valuable substances.

In Table 2, are collected further preferred formulations for use with the invention, including generic descriptions of compounds used in the formulation, the functions thereof, the preferred ranges, and actual amounts for an illustrative example of these compounds.

**Table 2**

| Component | Group | Function | * Preferred range | * Illustrative example |
|---|---|---|---|---|
| Metilparaben | Paraben | Antimicrobial preservative | 0.015 - 0.3 | 0.02 (0,04) |
| Propilparaben | Paraben | Antimicrobial preservative | 0.005 - 0.6 | 0.01 (0,02) |
| Cyclodextrin | Cyclic oligosaccharides | Solubilizing agent | 1 - 10 | 2.5 (5,0) |
| P 144 | Peptide | Drug or active principle | 0.01 - 0.09 | 0.01 (0.09) |
| Liquid paraffin | Mineral oil | Emollient, oleaginous vehicle, lubricant | 10 - 40 | 29.47 |
| Dimethicone 350 | Silicones (Dimethicone n: 250-400) | Emollient and antifoaming | 3 - 20 | 15 |
| Abil® EM 90 | Silicones (Dimethicone) | Surfactant w/o | 0.5 - 6 | 3 |
| purified water | - | Vehicle | q.s.p. 100 | q.s.p. 100 |

| | | | | |
|---|---|---|---|---|
| *weight/weight ratio (w/w); q.s.p.: quantum sufficit pro | | | | |

As such, in one particular embodiment the present invention relates to a pharmaceutical formulation comprising a TGF-β1 inhibitor peptide in an amount of 0.01% to 1% w/w, a β- or a γ-cyclodextrin in an amount of 1 to 10% w/w, cetyl PEG/PPG-10/1 dimethicone in an amount of 0.5% to 6% w/w, a preservative (e.g. a paraben, suchs as methylparabene, propylparabene, etc.) in an amount of 0.005% to 0.6% w/w, a silicone in an amount of 3% to 20% w/w, a mineral oil in an amount of 10% to 40% w/w, and water in q.s.p. 100%.

The emulsions of the present invention can be administered to a subject by any route of administration, topically, or as a buccal or nasal spray, ophthalmically, etc.

The complex of the invention comprising a TGF-β1 inhibitor peptide and a cyclodextrin or a derivative thereof, wherein
- the TGF-β1 inhibitor peptide is selected from a peptide having at least 70% sequence identity to one amino acid sequences of SEQ ID NO: 1-23; its salts, funtionally derivatives, variants, analogues and fragments thereof capable of inhibiting TGF-β1; and
- the cyclodextrin or a derivative thereof is selected from a β-cyclodextrin and a γ-cyclodextrin;
can be administered to a subject by any adequate route of administration, for example orally, rectally, parenterally, for example, intravenously, intramuscularly, or subcutaneously, etc., intracisternally, intravaginally, intraperitoneally, intravesically, topically, or by oronasal or pulmonary route , for example, as a buccal or nasal spray, ophthalmically, etc.

Suitable unit dosage forms that can be used in the present invention include, for example, hard gelatin capsules, soft gelatin capsules, tablets, caplets, enteric coated tablets, enteric coated hard gelatin capsules, enteric coated soft gelatin capsules, dragees, oral liquids, syrups, sprays, suppositories, etc.

In another aspect the present invention relates to the use of a complex of the invention, or the emulsion of the invention, or a pharmaceutical formulation [1] of the invention or the pharmaceutical formulation [2] of the invention, for the manufacture of a medicament for the treatment of a disease or condition mediated by TGF-β1. In another aspect, the present invention relates to a complex of the invention, or anemulsion of the invention, or a pharmaceutical formulation [1] or a pharmaceutical formulation [2] of the invention for use in the treatment of a disease or condition mediated by TGF-β1. Additionally, the present invention provides a method of treating a disease or condition mediated by TGF-β1 in a mammal in need of the treatment, said method comprising the administration to said mammal in need of the treatment of an effective amount of a complex of the invention, or an emulsion of the invention or a pharmaceutically formulation [1] of the invention or a pharmaceutical formulation [2] of the invention.

The term "a disease or condition mediated by TGF-β1" includes cardiovascular diseases such as hereditary hemorrhagic telangiectasia (Rendu-Osler-Weber syndrome), diseases of the aorta such as Loeys-Dietz syndrome, familial thoracic aortic aneurysm syndrome, and arterial tortuosity syndrome, primary pulmonary hypertension and familial pulmonary hypertension, pre-eclampsia, atherosclerosis, restenosis, hypertension, hypertrophic cardiomyopathy/congestive heart failure; connective tissue diseases such as Marfan syndrome and Marfan-like disorders, fibrotic diseases; skeletal and muscular disorders such as Camurati-Engelmann disease, fibrodysplasia ossificans progressiva, Hunter-Thompson and Grebe-type chondrodysplasias, osteoporosis, sclerosteosis and Van Buchem disease, brachydactyly and symphalangism, and Duchenne muscular dystrophy; reproductive disorders such as premature ovarian failure and persistent Müllerian duct syndrome; hereditary cancer syndromes such as juvenile polyposis syndrome, hereditary nonpolyposis colorectal cancer, and Bannayan-Riley-Ruvalcaba and Cowden syndromes; sporadic cancer such as breast cancer, colorectal cancer, pancreatic cancer, lung cancer, and prostate cancer; developmental disorders such as cleft palate and situs inversus and situs ambiguus.

In a particular embodiment, said diseases or conditions mediated by TGF-β1 include the fibroproliferative diseases. Specifically, fibroproliferative diseases include kidney disorders associated with unregulated TGF-β activity and excessive fibrosis including glomerulonephritis (GN), such as mesangial proliferative GN, immune GN, and crescentic GN. Other renal conditions include diabetic nephropathy, renal interstitial fibrosis, renal fibrosis in transplant patients receiving cyclosporin, and nephropathy associated with the immunodefiency human virus (HIV). Collagen vascular disorders include progressive systemic sclerosis, polymyositis, scleroderma, dermatomyositis, eosinophilic fascitis, morphea, or those associated with the occurrence of Raynaud's syndrome. Lung fibroses resulting from excessive TGF-β activity include adult respiratory distress syndrome, idiopathic pulmonary fibrosis, and interstitial pulmonary fibrosis often associated with autoimmune disorders, such as systemic lupus erythematosus and scleroderma, chemical contact, or allergies. Another autoimmune disorder associated with fibroproliferative characteristics is rheumatoid arthritis.

In a particular embodiment, said disease or pathological condition mediated by TGF-β1 is selected from hepatic fibrosis, pulmonary fibrosis, corneal fibrosis and fibrosis of peritoneal damage (e.g, fibrosis induced by peritoneal dialysis damage).

TGF-β superfamily members are continually being linked to other diseases. For example, alterations in TGF-β1 expression have been observed in several neurological disorders including autism, schizophrenia, Parkinson's disease, multiple sclerosis, and Alzheimer's disease, while activin expression is altered in Huntington's and Parkinson's disease. *TGFB1* polymorphisms are also associated with otosclerosis, a progressive loss of hearing that results from increased remodeling of the otic capsule, as well as in psoriases, biliary cirrhosis, and childhood asthma.

Further diseases or conditions mediated by TGF-β1 include keloids, hypertrophic scars such as those resulting from surgery or injury, chemical bums or thermal burns caused by heat or by cold, skin fibrosis associated with bone marrow transplantation, morphea, scleroderma and similar diseases such as acrokeratoelastoidosis; atrophoderma ofPasini and Pierini; CREST syndrome; dermatitis artefacta; diffuse scleroderma; eosinophilic fasciitis; graft-versus-host disease; keloid scleroderma; lichen sclerosus; linear scleroderma; limited systemic scleroderma; mandibuloacral dysplasia; skin changes associated with myeloma; nephrogenic fibrosing dermopathy; overlap syndrome; Parry-Romberg syndrome; porphyria cutanea tarda; progeria; skin changes of polyneuropathy syndrome, organomegaly, endocrinopathy, monoclonal proteins and skin changes or POEMS; pseudoscleroderma; Buschke scleroderma; scleromyxoedema; vitiligo; Werner syndrome, acne, cellulitis, Dupuytren syndrome, Peyronie disease, wrinkles and, in general, any skin lesion or pathology that passes through a stage with fibrosis or increase in the production and/or activation of TGF-β, as well as any disease with skin fibrosis as a complication.

Mammals eligible for the administration of the complex of the invention, the emulsion of the invention, or the pharmaceutical formulation [1] or [2] of the present invention include dogs, cats, horses, pigs, mice, rats, primates, and especially humans.

The following non-limiting examples help to illustrate the principles of the invention.

### EXAMPLES

### EXAMPLE 1

### Study of composition of the formulation

The purpose was to obtain a formulation having certain occlusive character, flowing properly in order to facilitate the industrial handling of big amounts and as in the case of the emulsion 965 (E.965) not allowing P144 to be absorbed and systemically distributed.

For obtaining the siliconic emulsion several surfactants were searched with the purpose of getting the finest possible emulsion. After a methodical search among different suppliers Abil^{®} Care 85 (Bis-PEG/PPG-16/16 PEG/PPG16/16) and Abil^{®} EM90 (cethyl PEG/PPG-10/1 dimethycone) were selected.

Several formulation assays were carried out until an stable, flowing emulsion with good organoleptic features was obtained. In table 3 the different assayed formulations are summarized.

**Table 3**

| **Composition of starting formulations to be assayed for the siliconic emulsion** | | | | | | | |
|---|---|---|---|---|---|---|---|
| **Formula** | **F.1** | **F.2** | **F.3** | **F.4** | **F.5** | **F.6** | **F.7** |
| **Component** | **(w/w)** | | | | | | |
| Methylparaben | 0.02 | 0.02 | 0.02 | 0.02 | 0.02 | 0.02 | 0.02 |
| Propylparaben | 0.01 | 0.01 | 0.01 | 0.01 | 0.01 | 0.01 | 0.01 |
| CD | 2.25 | 0.,8 | 0.8 | 0.8 | 1.6 | 2.5 | 2.5 |
| P144 | 0.01 | | | | | | |
| Paraffin | - | - | - | - | - | 30 | 29.47 |
| Dimethicone 350 | - | - | 56 | 28 | 15 | 15 | 15 |
| Dimethicone 1000 | 10 | 56 | - | 28 | - | - | - |
| Abil^{®} EM 90 | 5 | 2 | 2 | 2 | 2 | 3 | 3 |
| Sodium chloride | 2 | - | - | - | - | - | - |
| Abil^{®} care 85 | 35.75 | - | - | - | - | - | - |
| Water (q.s.) | 100 | 100 | 100 | 100 | 100 | 100 | 100 |

### [CD: Hydroxypropyl-beta-cyclodextrin]

The results obtained with the formulations shown in Table 3 were as follows:
- Formula F.1: as soon as the stirring of phase mixture was over the phases were separated. There was no formation of emulsion.
- Formula F.2: the emulsion was obtained and kept as such for few hours, but was separated after standing for some time.
- Formula F.3: the same occurred as in previous case, but it was separated after a bit longer standing period than in formula 2.
- Formula F.4: big differences were not noticed in respect to formulation 2. Formula F.5: the emulsion was not finally obtained.
- Formula F.6: a stable emulsion was obtained, with good fluidity and acceptable organoleptic properties. A part of it was preserved at room temperature and another part at 4°C.
- Formula F.7: final formulation was set to the parameters defined in the objects.

Therefore, formulation F.7 was selected as a starting point for transport or load capacity assays of the peptide (P144).

### EXAMPLE 2

### Process for manufacturing a siliconic emulsion loaded with P144 (100 µg/g)

The method for manufacturing the siliconic emulsion, according to formula F.7, was as follows:
1. Water was weighted.
2. Propylparaben and methylparaben were weighted and dissolved in water applying slight heat (lower than 35°C) in order to facilitate the dissolution of preservatives.
3. Once parabens are dissolved, the solution was left to cool off and then CD (hydroxypropyl-beta-ciclodextrin) was added little by little under stirring until complete dissolution.
4. Then, P144 was slowly added and under stirring, the time necessary for dissolving P144 was quite prolonged (one night).
5. In a different container, paraffin and dimethicone 350 were weighted over which Abil ^{®} EM 90 was added. The three components were mixed under magnetic stirring
6. Aqueous phase with P144 was added dissolved over oily phase until obtaining the emulsion. Once the maturing process of emulsion was over, three aliquotes of approximately 6 g were subjected to a test for checking the stability of the emulsion. Said test consisted on:
   - Centrifugating at 2,000 r.p.m. for 5 minutes
   - Centrifugating at 4,000 r.p.m. for 10 minutes
   - Centrifugating at 8,000 r.p.m. for 10 minutes
   - Centrifugating at 13,000 r.p.m. for 10 minutes
   - Heating at 90°C

In the first two cases, no alteration was observed in the aspect of the emulsion. In the third case, it was observed that a minimum amount of water was released onto the surface of the emulsion. However, no alteration was noticed after the emulsion was centrifuged at 13,000 r.p.m.or heated.

An alternative method was assayed for incorporating P144 in the formulation: the physical mixing. It consisted on dry mixing of P144 and the cyclodextrin with the aid of a mortar. Then this mixture was added over the water of the formulation resulting in a suspension which could not dissolve completely. For this reason, this way of incorporating P144 was discarded.

As manufacturing process was selected those in which a cyclodextrin solution was prepared over which a peptide was added, although a modification was incorporated, both phases in the emulsion were slightly warmed up (lower than 35°C).

### EXAMPLE 3

### Verification of the solution of P144 in the siliconic emulsion

A quick and simple method was searched allowing to know if P144 was dissolved in the formulation or on the contrary it was in a form of dispersed crystals in the emulsion. Because of the characteristics of P144, we decided that said method was the observation with the microscope of representative samples from the different assayed formulations by means of visible and UV light.

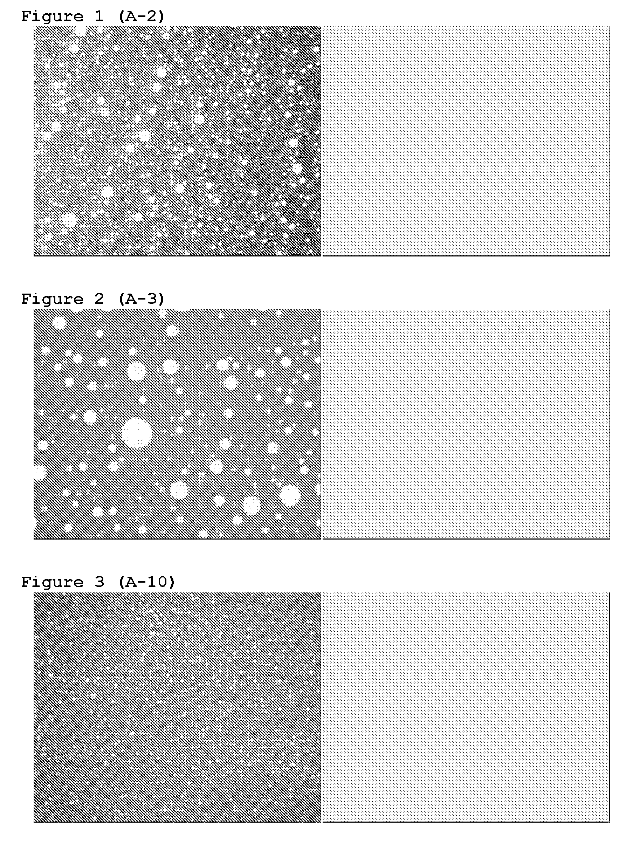

The experimental assayed conditions were as follows: microscope Nikon ECLIPSE E800, camera Nikon DIGITAL CAMERA DXM 1200, image analysis program ACT-1. The different samples were observed under visible light and UV radiation (EX 330-380, DM 400, BA 420) and at 4x. Figures 1-7 shows images of a part of batches manufactured with the above described formulation. Figures 1 and 2 show photograhs of the siliconic emulsion with 100 µg/g of P144 and the siliconic emulsion as a blank, without P144, respectively.

### EXAMPLE 4

### Comparison of the transport capacity of P144 between the emulsion E.965 and the new siliconic emulsion

The transport capacity of P144 between the emulsion E.965 and the new siliconic emulsion according to Formula F.7 initially selected was compared. Likewise, new formulations F.8 and F.9 were incorporated on which the amount of cyclodextrin was increased with the intention of parallelly increasing the load capacity of P144. These new formulations are provided in Table 4.

**Table 4**

| **Composition of the new siliconic emulsion, according to formula F.7 initially selected and the modified formula F.8 and F.9. The transport capacity of all 3 formula was assayed varying the load concentration of peptide P144 (see Table 5)** | | | |
|---|---|---|---|
| **Formula** | **F.7** | **F.8** | **F.9** |
| **Component** | **(w/w)** | | |
| Methylparaben | 0.02 | 0.04 | 0.04 |
| Propylparaben | 0.01 | 0.02 | 0.02 |
| CD | 2.5 | 4.2 | 5 |
| P144 | variable | variable | variable |
| Paraffin | 29.47 | 29.47 | 29.47 |
| Dimethicone | | | |
| 350 | 15 | 15 | 15 |
| Abil ^{®} EM 90 | 3 | 3 | 3 |
| Water (q.s.) | 100 | 100 | 100 |

Initially, an emulsion in which the amount of peptide was doubled reaching 200 µg/g (formula F.7) was prepared. This increase in mass was compensated by reducing the content of water, although it is an insignificant variation in the formulation as a whole. The process for obtaining the emulsion was the same as in the previous example.

In the microscopic assessment with visible and UV light of this formulation (batch A-4) no crystals of P144 were found which is indicative of a suitable solubilization of P144. Next, the same formulation F.7 and method were assayed but with a concentration of 300 µg/g; which is similar to the highest concentration achieved with the emulsion E.965. In the microscopic study of this formulation (batch A-5) with visible and UV light no crystals were detected.

Therefore, it can be concluded that a formulation with a P 144 load capacity similar to that of emulsion E.965 has been achieved, with better galenic properties, and wherein its manufacturing process requires providing considerably less heat.

From this point, any increase in the concentration of P144 in the formulation without detecting peptide crystals would be regarded as an improvement in the load capacity with respect to emulsion E.965 and it would suppose the achievement of the proposed objectives.

Using the same formulation F.7 as in previous cases new siliconic emulsions were prepared wherein the concentration of P144 was gradually increased: with 400 µg, 600 µg, 800 µg and 1,000 µg of P144 per gram of formulation. No P144 crystals were observed in the new four formulations when they were analyzed with visible or UV light (Figure 3).

However, later on new batches (A-12, A-14 and A-15) of the emulsion with P144 concentrations of 1,000 µg/g, 1,200 µg/g and 800 µg/g, respectively, were elaborated again. This time P144 crystals were clearly observed when analyzed with UV light.

The reason for these differences in the results among similar batches could be that the preparation of batches A-9 (800 µg/g) and A-10 (1,000 µg/g) was carried out with dilutions of a P144 solution previously prepared at a different concentration to the required one for any of these two batches.

A possible cause is that it could already be very close to the limit of solubility of P144 in the present formulation and therefore in some occasions a few crystals appear and in other occasions no crystals of P144 are observed.

In order to verify this possibility a new formula F.8 with slight modifications performed over the initial formulation F.7 was assayed. The content in hydroxypropyl-beta-cyclodextrin (CD) was increased from 2.5% w/w until 4.2% w/w and the content in parabens was doubled so that the new formulation was set as shown in Table 4.

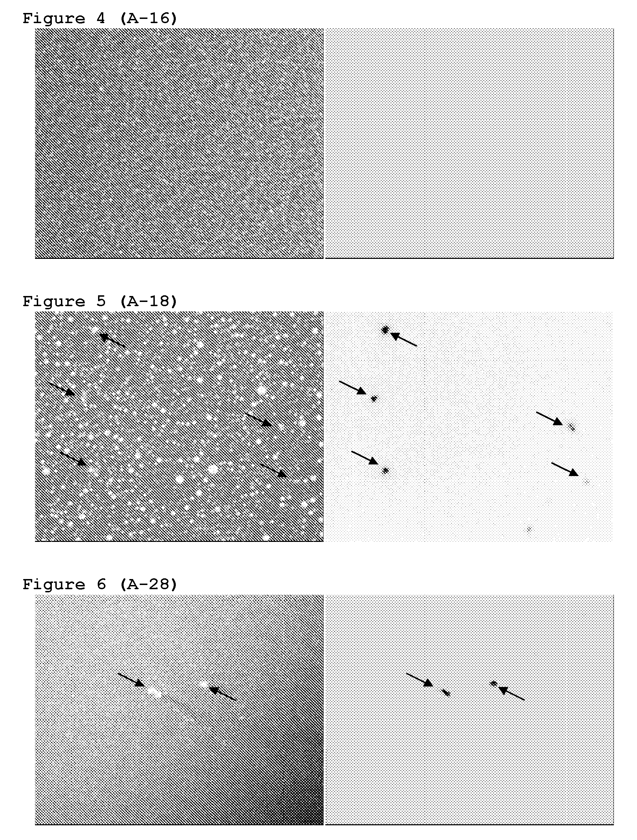

With this new alternative formulation a batch (A-16) with a load of 1000 µg/g of P144 was prepared. No P144 crystals were shown when observing the different fields in the microscope (Figure 4) suggesting that the second hypothesis was correct. By increasing the quantity of hydroxypropyl-beta-cyclodextrin (CD) a complete dissolution of the peptide was achieved.

This change in the formulation caused the necessity, according to the literature, of an increase in the preservatives in the formulation since it is well known that the presence of cyclodextrins reduces parabens efficiency. For this reason, the concentration of methyl and propylparaben was doubled.

Later on, having seen the cause of the occurrence of P144 crystals, it was decided to keep formulation F.7 initially assayed and adjust the amount of peptide able to uptake. In this way, new batches of the emulsion were prepared (A-17 and A-18) with a peptide concentration close to the limit of solubility: 750 µg/g and 700 µg/g. In both cases crystals were detected (Figure 5).

In view of these results, it was decided to set as a maximum concentration of P144 for this formulation F.7: 600 µg/g, designated as ESD600.

Table 5 summarizes the results obtained for the different assayed batches in the P144 load studies for the different formulations.

**Table 5**

| **Comparative summary of the results obtained (formation of crystals) for different emulsion batches prepared according to formula F.7, F.8 and F.9, varying load concentrations of peptide P144 (Examples 2, 4 and 5).** | | | | | |
|---|---|---|---|---|---|
| **Formula** | **P144** | | **Batch** | **Result** | |
| | µ**g/g** | **w/w** | | **Crystals** | **Figure** |
| F.7 | | | | | |
| | 0 | 0 | A-3 | | 2 |
| | 100 | 0.01 | A-2 | - | 1 |
| | 200 | 0.02 | A-4 | - | |
| | 300 | 0.03 | A-5 | - | |
| | 400 | 0.04 | A-6 | - | |
| | 600 | 0.06 | A-7 | - | |
| | 800 | 0.08 | A-9 | - | |
| | 1000 | 0.1 | A-10 | - | 3 |
| | | | | | |
| | 800 | 0.08 | A-15 | + | |
| | 1000 | 0.1 | A-12 | + | |
| | 1200 | 0.12 | A-14 | + | |
| | | | | | |
| | 700 | 0.07 | A-18 | + | 5 |
| | 750 | 0.075 | A-17 | + | |
| | | | | | |
| F.8 | | | | | |
| | 1000 | 0.1 | A-16 | - | 4 |
| | | | | | |
| F.9 | | | | | |
| | 0 | 0 | A-21 | - | |
| | 700 | 0.07 | A-29 | - | |
| | 800 | 0.08 | A-30 | - | 7 |
| | 1100 | 0.11 | A-28 | + | 6 |
| | 1200 | 0.12 | A-26 | + | |
| | 1400 | 0.14 | A-23 | + | |
| | | | | | |
| | 900 | 0.09 | A-31 | - | |

### Conclusions for the new siliconic emulsion of P144, formulation ESD600

It is considered that the objectives established at the beginning of this project were reached. The requirements initially disclosed for both formulations were:
- Allowing the local action of P144 without being significantly absorbed,
- Showing a good spreadability,
- Showing a pleasant appearance without being greasy.

With the new formulation ESD600 the following objectives were expected to be reached:
- facilitating solubilization of the active ingredient using any system which allows the removal/reduction of organic solvents,
- obtaining a more homogeneous and stable emulsion, and
- reducing the warming of the phases for obtaining the emulsion, without the risk for the active substance of loosing stability.

The first of the objectives was reached removing dimethyl sulfoxide (DMSO) as a solvent, replacing it with a solution of hydroxypropyl-beta-cyclodextrin which also allowed to double the highest concentration of peptide achieved with the emulsion E.965.

The second objective was also achieved. The third objective was also finally achieved, reducing warm-up temperature of the phases for the formation of the emulsion. While for preparing the emulsion E.965 work temperatures between 50-56°C were reached, for preparing the emulsion ESD600 the work temperature was kept always below 35°C.

### EXAMPLE 5

### Modification of the siliconic emulsion P144, formulation ESD600

A new objective for achieving a formulation capable of transporting an even higher peptide concentration was established.

Formulation F.7 (ESD600) was modified the least possible in order to increase the P144 load capacity as much as possible. For this the concentration of hydroxypropyl-beta-cyclodextrin was increased until 5% w/w and the concentration of parabens was also doubled, reducing proportionally the percentage of paraffin. In this way, the formulation would be as shown in Table 4, formula F.9 (ESD900). This emulsion would be the vehicle to transport considerably higher amounts of active ingredient. According to this new formula, different batches of emulsions loaded with increasing concentrations of P144: 0 µg/g (blank formulation), 700 µg/g, 800 µg/g, 1,100 µg/g, 1,200 µg/g y 1,400 µg/g, were prepared.

The blank formulation (ESD900 without P144) corresponding to the batch A-21, allowed to control that there was no signal neither with visible light nor with UV light.

**Table 6**

| **Composition of the siliconic emulsions for P144, ESD600 and ESD900** | | |
|---|---|---|
| **Formula** | **ESD600** | **ESD900** |
| **Component** | **w/w** | |
| Methylparaben | 0.02 | 0.04 |
| Propylparaben | 0.01 | 0.02 |
| CD | 2,5 | 5 |
| P144 | 0.01 a 0.06 | 0.01 a 0.09 |
| Paraffin | 29.47 | 29.47 |
| Dimethicone | | |
| 350 | 15 | 15 |
| Abil^{®} EM 90 | 3 | 3 |
| Water (q.s.) | 100 | 100 |

By observing with the microscope the corresponding samples, it was evidenced the formation of P144 crystals in the formulations with a concentration of P144 equal or higher than 1000 µg/g (Figures 5 and 6).

Later on, batch A-31 was prepared with a peptide concentration of 900 µg/g. In this case no P144 crystals were either observed.

Therefore, it could be concluded that with new formulation F.9 containing 5% w/w of hydroxypropyl-beta-cyclodextrin, the maximum concentration of P144 was 900 µg/g. This formulation was designated as ESD900.

### Conclusions

The siliconic emulsion of P144 (ESD600 or ESD900) allows the local action of P144 without being absorbed; shows a good spreadability and pleasant appearance without being greasy, being a more homogeneous and stable emulsion; facilitates the solubilization of the active ingredient using an aqueous solution of hydroxypropyl-beta-cyclodextrin (2.5% w/w in ESD600 or 5.0 w/w in ESD900) without needing organic solvents.

The capacity of P144 transport has been increased by duplicating (ESD600) or triplicating (ESD900) the load capacity of the emulsion E.965.

With the new siliconic emulsion the warming of the phases is reduced for obtaining the emulsion without the risk for the active substance of loosing stability.

### EXAMPLE 6

### Galenic characterization of the formulation ESD600

### 1. Macroscopic characteristics

Appearance: The siliconic emulsion ESD600 appeared uniform and milky, with a fluid consistency and excellent homogeneity.

### Odor: Odorless.

Tact: The siliconic emulsion ESD600 presented occlusive properties, with no drying effect, and left a slight slow-absorbing oily residue after application. The touch was smooth, with a bright appearance.

### 2. Microscopic characteristics

### 2.1. Droplet size:

Procedure: An aliquot of ESD600 siliconic emulsion was placed on a slide. Visualization was carried out under an Olympus CH40 microscope (x100 x 1.25 = x125).

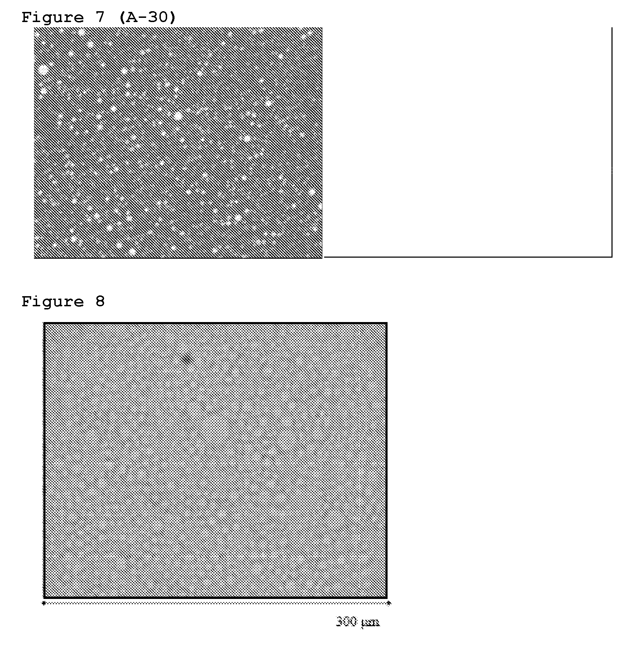

Result: Quite homogeneous droplet size, in the range of 2-3 µm, approximately 10% of the droplets measuring 10 µm in size. See Figure 8.

### 2.2. Determination of consistency

### - Measurement of spreading capacity (Figures 9-16):

### Procedure:

After marking the sides of a slide on millimetered paper and tracing the corresponding diagonals to obtain their intersection as reference central point, the slide was positioned taking care to ensure that it coincided with the aforementioned tracing, and about 25 mg of emulsion was placed at the central point. A second slide of known weight was then gently placed over the former slide, and after waiting for one minute, measurement was made of the two diameters of the circle formed with respect to the point of intersection of the traced diagonals, calculating the mean value and dividing by two. The mean radius of the circle was thus calculated. The same procedure was then repeated with three different weights, and always at intervals of one minute. The radii thus obtained were used to calculate the corresponding surfaces, based on the formula S = π*r² Spreading capacity was determined at room temperature. See Figures 9-16.

### Results:

Table 7 reports the weights used to determine the spreading capacity of the formulation, together with the corresponding diameter increments, mean radius and surface of the P144 siliconic emulsion ESD600 sample.

**Table 7**

| **Results of the spreading capacity tests of P144 siliconic emulsion ESD600** | | | | | | |
|---|---|---|---|---|---|---|
| | Weights (g) | Diameter A(mm) | Diameter B(mm) | Mean radius (mm) | Surface (mm²) | Surface increment (mm²) |
| Emulsion | 0 | 5 | 5 | 5 | 78.54 | 0 |
| Slide | 4.814 | 14 | 16 | 15 | 706.858 | 628.318 |
| 1 Weight (2.089 g) | 6.903 | 19 | 20 | 19.5 | 1194.59 | 1116.051 |
| 2 Weight (1.915 g) | 8.818 | 21 | 21 | 21 | 1385.44 | 1306.902 |
| 3 Weight (1.930 g) | 10.748 | 25 | 25 | 25 | 1963.5 | 1884.955 |

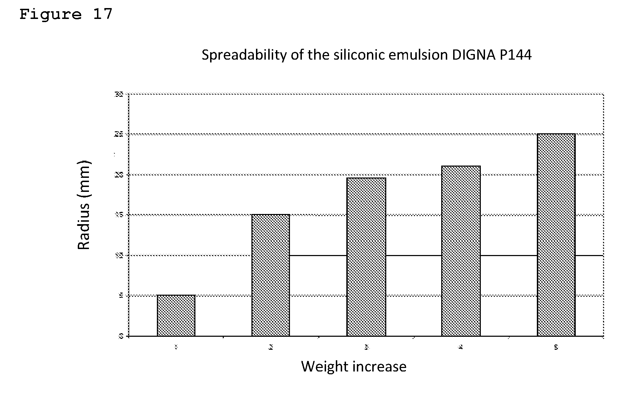

See Figure 17.

### The calculated surface increment was 1,884.955 mm².

The P144 siliconic emulsion ESD600 exhibited better spreading capacity than the emulsion E.965, since the surface increment was approximately twice as great.

### 3. Determination of the type of emulsion

Determination of the type of emulsion was carried out by two methods:

### 3.1. Dilution method: To a tube containing water (3 ml), we add a small amount of P144 siliconic emulsion ESD600, without stirring. If the external phase is aqueous, the water becomes turbid. If the external phase is oily, the water remains unaltered and does not become turbid.

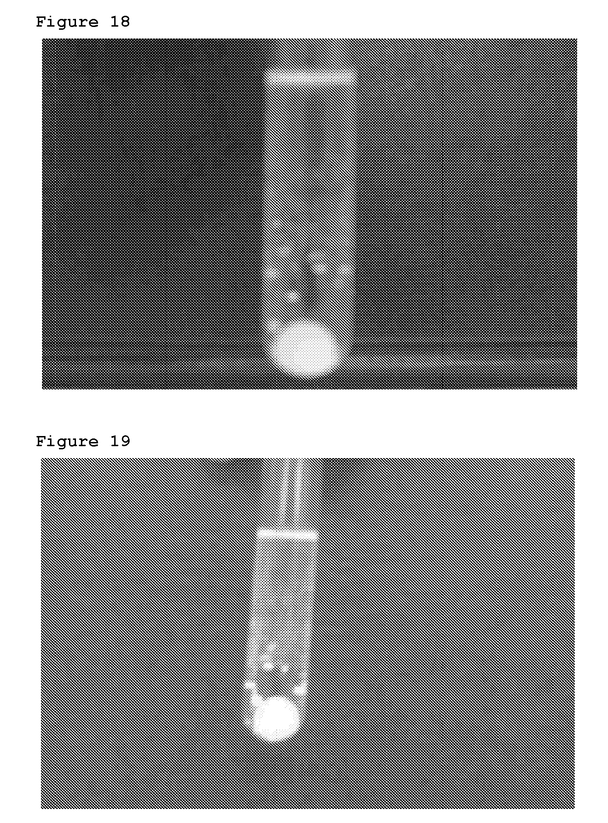

After performing the test as described, the water did not become turbid, and even oily droplets were seen; it therefore could be affirmed that the siliconic emulsion presented an oily external phase (w/o). Figures 18 and 19 show results obtained with this method.

### 3.2. Dye method: A water-soluble dye was used, specifically a solution of methylene blue (0.4% w/v). If the emulsion is of the o/w type, the dye will disperse. However, if the emulsion is of type w/o, it will be repelled and will not disperse.

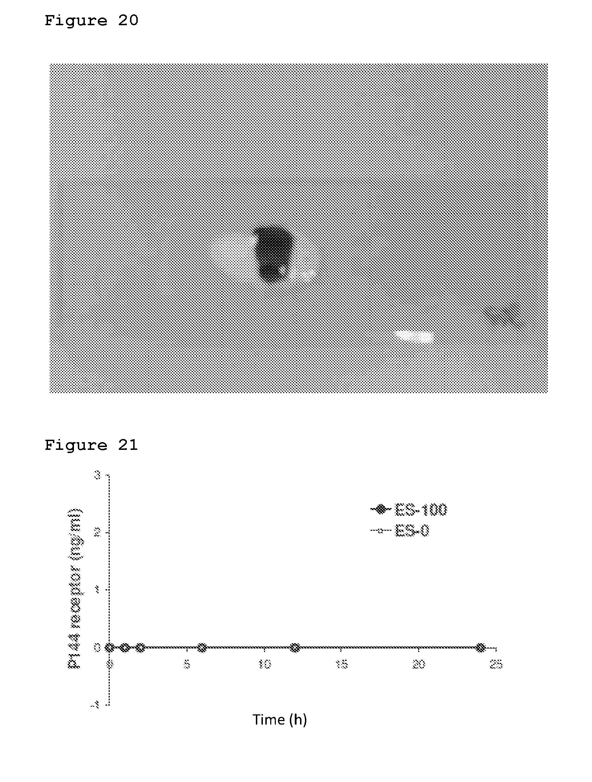

A small amount of the study emulsion was placed on a slide. A drop of methylene blue was then added, without mixing. After performing the test as described, the emulsion was seen to repel the dye; we therefore concluded that it was of type w/o. See Figure 20.

### 4. Determination of pH

Determination of the pH value was carried out by diluting 500 mg of emulsion in 10 ml of water, followed by filtration. Three measurements were made with a GLP 21 Crison pHmeter, with the following results:
- Sample no. 1: 5.78
- Sample no. 2: 5.79
- Sample no. 3: 5.70
- mean pH = 5.76 ± 0.049

### EXAMPLE 7

### Study of the percutaneous absorption of P144 formulated in the emulsion ESD600

### Planning and objective

This study had as an objective assessing the absorption and percutaneous penetration in samples of pig ear skin of the peptide P144 formulated in the formulation designated as "ESD600" at a concentration of 100 µg/g (ESD600_{[100]}). As a reference product it was used the formulation "ESD600 control" without peptide (ESD600_{[0]}).

### Materials and methods

The pig ear skin was acquired from hybrid pigs for human consumption. Ears were treated as disclosed later in "Process 1. Step 1: Dermatomization".

The assay product was the emulsion ESD600 with 100 µg P144/g (ESD600_{[100]}, No. batch: X-6). This semisolid preparation was carried out according to the above mentioned process. Its composition is shown on Table 8.

**Table 8**

| **Components** | **Manufacturer** | **Reference** | **Percentage** | **Quantity** |
|---|---|---|---|---|
| Dimethicone 350 | Roig-Farma | 301119-09 | 15.0% | 75.0 |
| Paraffin | Roig-Farma | 31629-09 | 29.5% | 147.5 |
| Methylparaben | Roig-Farma | 31169-24 | 0.02% | 0.1 |
| Propylparaben | Roig-Farma | 31171-24 | 0.01% | 0.05 |
| Abil^{®} EM 90 | Goldschmidt | 00201109 | 3.00% | 15 |
| CD | Aldrich | 33,260-7 | 2.5% | 12.5 |
| Peptid P144 | | | 0.01% | 0.05 |
| Purified water | | | 100.0% | q.s. 500g. |

### CD: Hydroxypropyl-beta-cyclodextrin.

The reference product was the control siliconic emulsion ESD600_{[0]} without P144 (ESD600_{[0]}, No. batch: X-5). This semisolid preparation was carried out according to the above mentioned process, without adding the active ingredient. Its composition is shown on Table 9.

**Table 9**

| **Components** | **Manufacturer** | **Reference** | **Percentage** | **Quantity** |
|---|---|---|---|---|
| Dimethicone 350 | Roig-Farma | 301119-09 | 15.0% | 75.0 |
| Paraffin | Roig-Farma | 31629-09 | 29.5% | 147.5 |
| Methylparaben | Roig-Farma | 31169-24 | 0.02% | 0.1 |
| Propylparaben | Roig-Farma | 31171-24 | 0.01% | 0.05 |
| Abil ^{®} EM 90 | Goldschmidt | 00201109 | 3.00% | 15 |
| CD | Aldrich | 33,260-7 | 2.5% | 12.5 |
| Purified water | | | 100.0% | q.s. 500 g. |

### CD: Hydroxypropyl-beta-cyclodextrin.

Kit used:
- Balance Mettler AT261 Delta Range
- pHmeter Crison, pH meter GLP 21
- Franz Variomag Telesystem diffusion cell. Franz cell device consisting of 6 cells made of
   borosilicate glass and teflon with a receptor chamber (volume 7 mL) and a donor chamber (diameter 15 mm).
- Dermatomo (Aesculap-Wagner dermatome C. GA 630; B. Braun Surgical S.A., Barcelona, Spain)
- Arch at-80°C (Forma Scientific, 938)
- Freezer at -20°C

All chemical reagents, salts and products had analytical quality. Phosphate buffered solution (PBS) (Sigma, ref: P4417- 100 TAB), hydroxypropyl-beta-cyclodextrin (OH-CD), (Sigma, ref. 332607), Tissue Tek® OCT Compound (Sakura, ref: 4583). Water type II, obtained in a system Wasserlab (Automatic Model N° AU 050503) was used to prepare the solutions.

Vials for collecting samples from the cells (Watters Ref.: 7176).

### Criotubes for preserving samples

The device "Franz diffusion cells" consists of 6 cells, distributed in 2 rows. Each cell has 2 chambers: donor and receptor. The product to be assayed or its control is placed in the first chamber and the solution which will capture the amount of drug crossing through the experimental system (pig or human skin) is placed in the second chamber. This experimental system is found in the interface between the donor chamber and the receptor chamber. The distribution of products to be assayed in the device "Franz Cells" was carried out as follows: ESD600_{[100]} (Cells 5 and 6), ESD600_{[0]} Control (Cell 2). The experiment with pig ear skin was repeated 4 times.

Process

### 1. Step 1: Dermatomization

The same day as obtained, the pig ears were washed, treated and cut with the aid of a dermatome in order to obtain membranes having 1 mm thickness and a minimum surface of 4 cm². The membranes were stored separately in a freezer at -20°C.

### 2. Step 2: Percutaneous diffusion assay.

### 2.1. Percutaneous diffusion assay

A 24 hours (h) diffusion study was carried out wherein the siliconic emulsion ESD600_{[100]} with 100 µg/g of peptide was assayed. As a diffusion membrane dermatomized pig skin ear at 1.0 mm was used. Before starting the assay, skins were hydrated by immersion for 30 minutes with PBS (phosphate buffered solution, pH 7.4±0.2). On the other hand, the receptor of each of the cells was filled with 7 ml of phosphate buffered solution containing OH-CD 5% w/w set at pH 7.4±0.2. The portion of dermatomized and hydrated skin was placed onto the receptor chamber. On this skin, the receptor chamber was fixed (15 mm diameter), wherein about 0.9 g of semisolid preparation (1 ml volume), product to be assayed (ESD600_{[100]}) or its reference product (control Siliconic Emulsion ESD600; ESD600_{[0]}) was placed. The receptor was kept at a temperature of 32.5 ± 0.5°C and under stirring at 400 r.p.m. At fixed time intervals (0h, 1h, 2h, 6h, 12h and 24h) 1 ml samples were manually collected from the receptor chamber, replacing then this volume with 1 ml of original solution in order to keep the receptor volume constant. The collected samples were stored in criotubes at -80°C in a -80°C freezer for the subsequent chromatographic analysis.

### 3. Study of the presence of P 144 in the different layers of skin

Once the assay is over, membrane cuts were carried out. For this, the skin was previously removed from the cells and remaining products to be assayed or control were removed washing with water type II. The samples were placed onto a hard surface and an enough amount of OCT was added so as to obtain an homogeneous frozen block which allows to perform the consecutive cuts. Over each sample six consecutive cuts were made with the aid of a criostate, in depth, of 50 µm for pig ear skin. These cuts were collected individually in criotubes, identified with a code and eventually stored at -80°C until the subsequent chromatographic analysis. Tables 10-13 summarize the characteristics of skin samples. Said tables show the percentage of skin surface (needed for quantifying the P144 thereof) analysed after making the cut in the criostate.

**Table 10**

| **Quantification in pig ear skin cuts. Cut surface (S= 176 mm²; thickness = 50 mm). Experiment 1** | | | |
|---|---|---|---|
| **Sample name** | **Description** | **Surface cut (%)** | **Concentration (**µ**g/cm³)** |
| 1.2.1 | Exp:1 Cell 2 (ESD600_{[100]}) Cut 1 | 20 | 19.563 |
| 1.2.2 | Exp:1 Cell 2 (ESD600_{[100]}) Cut 2 | 40 | 3.706 |
| 1.2.3 | Exp:1 Cell 2 (ESD600_{[100]}) Cut 3 | 50 | 2.556 |
| 1.2.4 | Exp:1 Cell 2 (ESD600_{[100]}) Cut 4 | 70 | 3.741 |
| 1.2.5 | Exp:1 Cell 2 (ESD600_{[100]}) Cut 5 | 70 | 0.808 |
| 1.2.6 | Exp:1 Cell 2 (ESD600_{[100]}) Cut 6 | 80 | 0.856 |
| 1.5.1 | Exp:1 Cell 5 (ESD600_{[100]}) Cut 1 | 30 | 1.994 |
| 1.5.2 | Exp:1 Cell 5 (ESD600_{[100]}) Cut 2 | 40 | 1.045 |
| 1.5.3 | Exp:1 Cell 5 (ESD600_{[100]}) Cut 3 | 60 | 0.702 |
| 1.5.4 | Exp:1 Cell 5 (ESD600_{[100]}) Cut 4 | 60 | 0.620 |
| 1.5.5 | Exp:1 Cell 5 (ESD600_{[100]}) Cut 5 | 80 | 0.438 |
| 1.5.6 | Exp:1 Cell 5 (ESD600_{[100]}) Cut 6 | 90 | 0.398 |
| 1.6.1 | Exp:1 Cell 6 (ESD600_{[100]}) Cut 1 | 40 | <LOQ |
| 1.6.2 | Exp:1 Cell 6 (ESD600_{[0]}) Cut 2 | 30 | <LOQ |
| 1.6.3 | Exp:1 Cell 6 (ESD600_{[0]}) Cut 3 | 50 | <LOQ |
| 1.6.4 | Exp:1 Cell 6 (ESD600_{[0]}) Cut 4 | 80 | <LOQ |
| 1.6.5 | Exp:1 Cell 6 (ESD600_{[0]}) Cut 5 | 80 | <LOQ |
| 1.6.6 | Exp:1 Cell 6 (ESD600_{[0]}) Cut 6 | 90 | <LOQ |

| | | | |
|---|---|---|---|
| LOQ: Limit of quantification | | | |

**Table 11**

| **Quantification in pig ear skin cuts. Cut surface (S= 176 mm²; thickness = 50 mm). Experiment 2** | | | |
|---|---|---|---|
| **Sample name** | **Description** | **Surface cut (%)** | **Concentration (**µ**g/cm³)** |
| 2.2.1 | Exp:2 Cell 2 (ESD600_{[100]}) Cut 1 | 30 | 8.498 |
| 2.2.2 | Exp:2 Cell 2 (ESD600_{[100]}) Cut 2 | 40 | 0.799 |
| 2.2.3 | Exp:2 Cell 2 (ESD600_{[100]}) Cut 3 | 30 | 24.198 |
| 2.2.4 | Exp:2 Cell 2 (ESD600_{[100]}) Cut 4 | 50 | 11.017 |
| 2.2.5 | Exp:2 Cell 2 (ESD600_{[100]}) Cut 5 | 70 | 8.429 |
| 2.2.6 | Exp:2 Cell 2 (ESD600_{[100]}) Cut 6 | 80 | 8.588 |
| 2.5.1 | Exp:2 Cell 5 (ESD600_{[100]}) Cut 1 | 30 | 0.795 |
| 2.5.2 | Exp:2 Cell 5 (ESD600_{[100]}) Cut 2 | 40 | 15.067 |
| 2.5.3 | Exp:2 Cell 5 (ESD600_{[100]}) Cut 3 | 40 | 1.940 |
| 2.5.4 | Exp:2 Cell 5 (ESD600_{[100]}) Cut 4 | 70 | 3.881 |
| 2.5.5 | Exp:2 Cell 5 (ESD600_{[100]}) Cut 5 | 70 | 12.286 |
| 2.5.6 | Exp:2 Cell 5 (ESD600_{[100]}) Cut 6 | 80 | 7.660 |
| 2.6.1 | Exp:2 Cell 6 (ESD600_{[0]}) Cut 1 | 30 | <LOQ |
| 2.6.2 | Exp:2 Cell 6 (ESD600_{[0]}) Cut 2 | 50 | <LOQ |
| 2.6.3 | Exp:2 Cell 6 (ESD600_{[0]}) Cut 3 | 80 | <LOQ |
| 2.6.4 | Exp:2 Cell 6 (ESD600_{[0]}) Cut 4 | 90 | <LOQ |
| 2.6.5 | Exp:2 Cell 6 (ESD600_{[0]}) Cut 5 | 90 | <LOQ |
| 2.6.6 | Exp:2 Cell 6 (ESD600_{[0]}) Cut 6 | 95 | <LOQ |

**Table 12**

| **Quantification in pig ear skin cuts. Cut surface (S= 176 mm²; thickness = 50 mm). Experiment 3** | | | |
|---|---|---|---|
| **Sample name** | **Description** | **Surface cut (%)** | **Concentration (**µ**g/cm³)** |
| 3.2.1 | Exp:3 Cell 2 (ESD600_{[100]}) Cut 1 | 20 | 136.798 |
| 3.2.2 | Exp:3 Cell 2 (ESD600_{[100]}) Cut 2 | 30 | 37.633 |
| 3.2.3 | Exp:3 Cell 2 (ESD600_{[100]}) Cut 3 | 40 | 45.961 |
| 3.2.4 | Exp:3 Cell 2 (ESD600_{[100]}) Cut 4 | 80 | 24.637 |
| 3.2.5 | Exp:3 Cell 2 (ESD600_{[100]}) Cut 5 | 60 | 29.884 |
| 3.2.6 | Exp:3 Cell 2 (ESD600_{[100]}) Cut 6 | 70 | 37.251 |
| 3.5.1 | Exp:3 Cell 5 (ESD600_{[100]}) Cut 1 | 30 | 13.863 |
| 3.5.2 | Exp:3 Cell 5 (ESD600_{[100]}) Cut 2 | 50 | 0.584 |
| 3.5.3 | Exp:3 Cell 5 (ESD600_{[100]}) Cut 3 | 100 | 12.716 |
| 3.5.4 | Exp:3 Cell 5 (ESD600_{[100]}) Cut 4 | 20 | 1.308 |
| 3.5.5 | Exp:3 Cell 5 (ESD600_{[100]}) Cut 5 | 70 | 10.097 |
| 3.5.6 | Exp:3 Cell 5 (ESD600_{[100]}) Cut 6 | 70 | 11.002 |
| 3.6.1 | Exp:3 Cell 6 (ESD600_{[0]}) Cut 1 | 40 | <LOQ |
| 3.6.2 | Exp:3 Cell 6 (ESD600_{[0]}) Cut 2 | 50 | <LOQ |
| 3.6.3 | Exp:3 Cell 6 (ESD600_{[0]}) Cut 3 | 50 | <LOQ |
| 3.6.4 | Exp:3 Cell 6 (ESD600_{[0]}) Cut 4 | 60 | <LOQ |
| 3.6.5 | Exp:3 Cell 6 (ESD600_{[0]}) Cut 5 | 60 | <LOQ |
| 3.6.6 | Exp:3 Cell 6 (ESD600_{[0]}) Cut 6 | 60 | <LOQ |

**Table 13**

| **Quantification in pig ear skin cuts. Cut surface (S= 176 mm²; thickness = 50 mm). Experiment 4** | | | |
|---|---|---|---|
| **Sample name** | **Description** | **Surface cut (%)** | **Concentration (**µ**g/cm³)** |
| 4.2.1 | Exp:4 Cell 2 (ESD600_{[100]}) Cut 1 | 50 | 31.850 |
| 4.2.2 | Exp:4 Cell 2 (ESD600_{[100]}) Cut 2 | 50 | 25.606 |
| 4.2.3 | Exp:4 Cell 2 (ESD600_{[100]}) Cut 3 | 70 | 24.540 |
| 4.2.4 | Exp:4 Cell 2 (ESD600_{[100]}) Cut 4 | 80 | 21.279 |
| 4.2.5 | Exp:4 Cell 2 (ESD600_{[100]}) Cut 5 | 80 | 26.482 |
| 4.2.6 | Exp:4 Cell 2 (ESD600_{[100]}) Cut 6 | 70 | 31.855 |
| 4.5.1 | Exp:4 Cell 5 (ESD600_{[100]}) Cut 1 | 30 | 149.132 |
| 4.5.2 | Exp:4 Cell 5 (ESD600_{[100]}) Cut 2 | 30 | 156.107 |
| 4.5.3 | Exp:4 Cell 5 (ESD600_{[100]}) Cut 3 | 70 | 76.175 |
| 4.5.4 | Exp:4 Cell 5 (ESD600_{[100]}) Cut 4 | 70 | 124.940 |
| 4.5.5 | Exp:4 Cell 5 (ESD600_{[100]}) Cut 5 | 60 | 68.073 |
| 4.5.6 | Exp:4 Cell 5 (ESD600_{[100]}) Cut 6 | 50 | 13.699 |
| 4.6.1 | Exp:4 Cell 6 (ESD600_{[0]}) Cut 1 | 20 | <LOQ |
| 4.6.2 | Exp:4 Cell 6 (ESD600_{[0]}) Cut 2 | 30 | <LOQ |
| 4.6.3 | Exp:4 Cell 6 (ESD600_{[0]}) Cut 3 | 40 | <LOQ |
| 4.6.4 | Exp:4 Cell 6 (ESD600_{[0]}) Cut 4 | 40 | <LOQ |
| 4.6.5 | Exp:4 Cell 6 (ESD600_{[0]}) Cut 5 | 100 | <LOQ |
| 4.6.6 | Exp:4 Cell 6 (ESD600_{[0]}) Cut 6 | 10 | <LOQ |

### Treatment, assessment and interpretation of results

The quantification of peptide P144, both in skin samples and reservoirs, was carried out by high performance liquid chromatography with mass detection (HPLC/MS/MS).

### Sample from receptors:

The limit of quantification (LOQ) of the technique HPLC-Mass for samples from receptors is 10 ng/ml, and has been used to make one of the following statements:
- When amounts of peptide P144 are not detected, in the collected solutions of the receptor chamber (negative samples or in any case lower than the limit of quantification of the analytical technique): "P144, formulated in the semisolid preparation ESD600, is not able to cross through the cutaneous barrier".
- When amounts of peptide P144 are detected, in the collected solutions of the receptor chamber, higher than the limit of quantification of the analytical technique: "P144, formulated in the semisolid preparation ESD600, is able to cross through the cutaneous barrier"

If amounts of peptide P144 had been detected in the receptor chamber, representations X-Y of accumulative amounts versus time would have been carried out. Likewise, in the case of the presence of a significant amount of drug, the transdermal flow (J) from slope of the curve representing the steady state would have been calculated.

### Samples from horizontal cuts of skin:

In the study of the presence of P144 in the different layers (depths) of the skin, the quantity of P144 quantified by HPLC-MS was represented versus depth. All data were represented as the mean and standard deviation, showing the number of repetitions (n). The existence of significant differences and statistical comparisons was analyzed by ANOVA. For these comparisons between different treatments, Tukey's test and Dunnett's test were applied. In any other case, p<0.05 was considered as significant. The statistical analysis was carried out using the program SPSS version 11.

### Results and discussion over cutaneous absorption and penetration study on pig ear skin

Figure 21 shows the amount of P144, formulated in ESD600 with 100 µg P144/g (ESD600_{[100]}), able to achieve the receptor in the transdermal absorption study. As shown the amount of P144 detected in the receptor was always lower than 2 ng in all analyzed cases (limit of quantification of the technique HPLC/MS/MS; see Table 14). At any case, it was not possible to see a signal which permitted to venture the pass of a minimum peptide fraction. Because of this, it can be stated that, under the conditions of this study, there is no transdermal flow of P 144 through the pig ear skin towards the general circulation.

**Table 14**

| **Table with results of quantification of receptors** | |
|---|---|
| **Sample name** | **CONC. (ng/ml)** |
| 1.2 t=0h | < LOQ |
| 1.2 t=1h | < LOQ |
| 1.2 t=2h | < LOQ |
| 1.2 t=6h | < LOQ |
| 1.2 t=12h | < LOQ |
| 1.2 t=24h | < LOQ |
| 1.5 t=0h | < LOQ |
| 1.5 t=1h | < LOQ |
| 1.5 t=2h | < LOQ |
| 1.5 t=6h | < LOQ |
| 1.5 t=12h | < LOQ |
| 1.5 t=24 h | < LOQ |
| 1.6 t=0h | < LOQ |
| 1.6 t=1h | < LOQ |
| 1.6 t=2h | < LOQ |
| 1.6 t=6h | < LOQ |
| 1.6 t=12h | < LOQ |
| 1.6 t=24h | < LOQ |
| 2.2 t=0h | < LOQ |
| 2.2 t=1h | < LOQ |
| 2.2 t=2h | < LOQ |
| 2.2 t=6h | < LOQ |
| 2.2 t=12h | < LOQ |
| 2.2 t=24 h | < LOQ |
| 2.5 t=0h | < LOQ |
| 2.5 t=1h | < LOQ |
| 2.5 t=2h | < LOQ |
| 2.5 t=6h | < LOQ |
| 2.5 t=12h | < LOQ |
| 2.5 t=24 h | < LOQ |
| 2.6 t=0h | < LOQ |
| 2.6 t=1h | < LOQ |
| 2.6 t=2h | < LOQ |
| 2.6 t=6h | < LOQ |
| 2.6 t=12h | < LOQ |
| 2.6 t=24 h | < LOQ |
| 3.2 t=0h | < LOQ |
| 3.2 t=1h | < LOQ |
| 3.2 t=2h | < LOQ |
| 3.2 t=6h | < LOQ |
| 3.2 t=12h | < LOQ |
| 3.2 t=24h | < LOQ |
| 3.5 t=0h | < LOQ |
| 3.5 t=1h | < LOQ |
| 3.5 t=2h | < LOQ |
| 3.5 t=6h | < LOQ |
| 3.5 t=12h | < LOQ |
| 3.5 t=24 h | < LOQ |
| 3.6 t=0h | < LOQ |
| 3.6 t=1h | < LOQ |
| 3.6 t=2h | < LOQ |
| 3.6 t=6h | < LOQ |
| 3.6 t=12h | < LOQ |
| 3.6 t=24 h | < LOQ |
| 4.2 t=0h | < LOQ |
| 4.2 t=1h | < LOQ |
| 4.2 t=2h | < LOQ |
| 4.2 t=6h | < LOQ |
| 4.2 t=12h | < LOQ |
| 4.2 t=24 h | < LOQ |
| 4.5 t=0h | < LOQ |
| 4.5 t=1h | < LOQ |
| 4.5 t=2h | < LOQ |
| 4.5 t=6h | < LOQ |
| 4.5 t=12h | < LOQ |
| 4.5 t=24 h | < LOQ |
| 4.6 t=0h | < LOQ |
| 4.6 t=1h | < LOQ |
| 4.6 t=2h | < LOQ |
| 4.6 t=6h | < LOQ |
| 4.6 t=12h | < LOQ |
| 4.6 t=24 h | < LOQ |

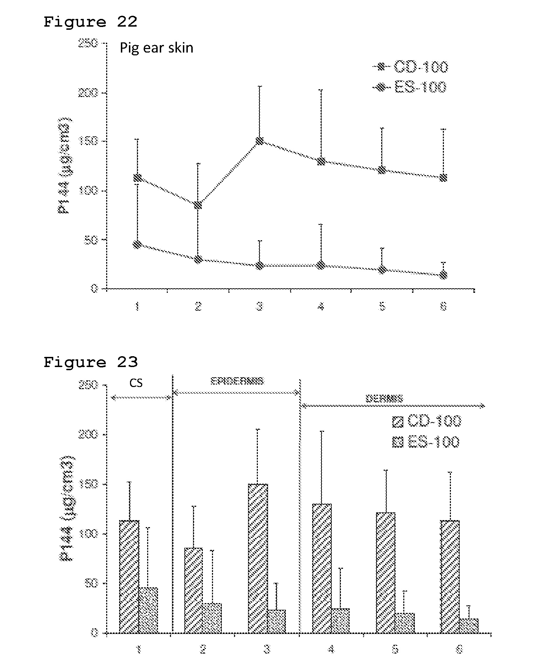

Tables 10-13 show the results for the determination of P144 in the different analyzed layers of the pig skin. Figure 22 shows the distribution of P 144 in the pig ear skin after topically applying ESD600_{[100]} for 24 h and compares it to the results obtained with the emulsion 965 (E.965) containing the same amount of peptide (ESD600_{[100]}). This distribution was estimated by means of the horizontal cut of portions of skin of 50 mm. As a control, skin cuts treated with ESD600_{[0]}, without P144, were used. At any case, no significant differences were found in the content of the drug in the 6 performed cuts (p<0.05). In case of the control emulsion ESD600_{[0]} no amounts of P144 were found (LOQ: 100 ng).

From a structural point of view, pig skin seems to be the most similar to human skin (Touitou et al.. J. Controlled Release, 1998, 56, 7-21; Simon and Maibach. Skin Pharmacol. Appl. Skin Physiol. 2000, 13, 229-234). Furthermore, it is disclosed the suitability for permeability study of many dugs through skin (Neubert and Wohlrab. Acta Pharm. Technol., 1990, 36, 197-206). Jenning et al. (Jenning et al. Eur. J. Pharm. Biopharm., 2000, 49, 211-218) has established, by means of optical microscopy, that in the pig skin a stratum corneum layer of approximately 100 mm, followed by a viable epidermis layer of 100-200 mm can be distinguished. Dermis would be located between 200 and 500 mm, considering the remaining part dermis and small portions of subcutaneous fat tissue (Jenning et al. Eur. J. Pharm. Biopharm., 2000, 49, 211-218).

Results from distribution of P144 in pig ear skin (Figure 23) show clearly the affinity of P 144 for the different areas thereof. In the case of ESD600 and opposite to the results obtained with E.965, however, this affinity is higher for the stratum corneum (SC) of skin. Actually, a concentration gradient of P144 can be observed from stratum corneum until the last layer of the dermis.

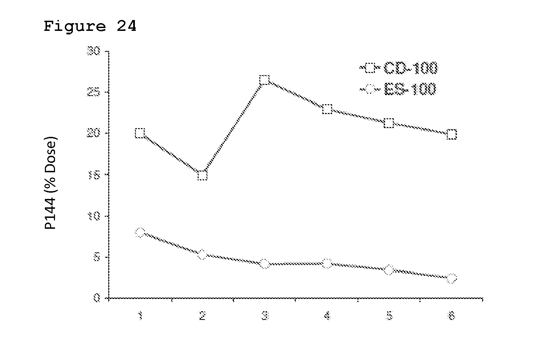

With data about cutaneous penetration, the theoretical quantity of the dose fraction existing in the different layers of skin has been calculated. Figure 24 shows these results for ESD600 and are compared with those obtained for E.965. As can be shown, the siliconic emulsion ESD600 has a significantly lower effect than emulsion E.965 for enhancing the penetration of P144 in the different layers of the skin.

### EXAMPLE 8

### Solubility study of P144

### Preparation of the optimized peptide product

1. Human albumin-peptide lyophilized samples: 2 vials, each one contained lyophilized product of 500µg of the peptide and 400 mg of Human albumin.
2. Human albumin-peptide lyophilized samples: 2 vials, each one contained lyophilized product of 500µg of the peptide and 200 mg of Human albumin.
3. Hycroxy-propyl-β-cyclodextrins-peptide lyophilized samples: 2 vials, each one contained lyophilized product of 500µg of the peptide and 100 mg of hydroxy-propyl-β-cyclodextrins.
4. Urea-peptide lyophilized samples: 2 vials, each one contained lyophilized product of 500µg of the peptide and 200 mg of urea.

The original volume before lyophilization was 2 ml. We recommend dispersing each vial with 2 ml of distilled water.

**Table 15**

| **Solvent** | **Solubility** |
|---|---|
| distilled water | insoluble |
| PEG 400 | insoluble |
| Ethanol | insoluble |
| Glycerol | insoluble |
| Pluronic F68 (1-10%) | insoluble |
| Dimethyl sulfoxide (DMSO) | very soluble |
| Tetrahydrofurane (THF) | insoluble |
| Hexane | insoluble |
| Dimethylformamide (DMF) | insoluble |
| Dicloromethane (DCM) | insoluble |
| PLASDONE 5% | insoluble |
| Proplylene glycol | insoluble |
| Gantrez ^{®}S97 2-5% | insoluble |
| Sodium deoxycholate 5% | insoluble |
| Urea 10% | soluble (250 µg/mL) |
| Human albumin 20% | soluble (250 µg/mL) |
| Hydroxy-propyl-β-cyclodextrin 5% | soluble (250 µg/mL) |

## Claims

1. A complex comprising a TGF-β1 inhibitor peptide and a cyclodextrin or a derivative thereof, wherein
• the TGF-β1 inhibitor peptide is selected from a peptide having at least 70% sequence identity to one amino acid sequences of SEQ ID NO: 1-23, its salts, functional derivatives, variants, analogs, or fragments thereof with capacity to inhibit TGF-β1; and
• the cyclodextrin or a derivative thereof is selected from a β-cyclodextrin and a y-cyclodextrin.

2. The complex according to claim 1, wherein the β-cyclodextrin is a mono-or poly(C₁C₆) hydroxyalkylated β-cyclodextrin.

3. The complex according to any one of claims 1 or 2, wherein the TGF-β1 inhibitor peptide is present in a weight ratio of about 0.002:1 to about 0.024:1 relative to the cyclodextrin.

4. A pharmaceutical formulation comprising the complex according to any one of claims 1 to 3, wherein the TGF-β1 inhibitor peptide is present in a therapeutically effective amount and the cyclodextrin is a pharmaceutically acceptable cyclodextrin.

5. Pharmaceutical formulation according to claim 4 further comprising cetyl PEG/PPG-10/1 dimethicone.

6. A TGF-β1 inhibitor peptide emulsion comprising cetyl PEG/PPG-10/1 dimethicone.

7. Emulsion according to claim 6, wherein the TGF-β1 inhibitor peptide is selected from a peptide having at least 70% sequence identity to the amino acid sequences of SEQ ID NO: 1-23.

8. Emulsion according to claim 7, wherein the TGF-β1 inhibitor peptide forms a complex with a cyclodextrin or a derivative thereof.

9. Emulsion according to claim 8, wherein the TGF-β1 inhibitor peptide is present in a weight ratio of about 0.002:1 to about 0.024:1 relative to the cyclodextrin.

10. A pharmaceutical formulation comprising a TGF-β1 inhibitor peptide emulsion according to any one of claims 6 to 9, wherein the TGF-β1 inhibitor peptide is present in a therapeutically effective amount, and the cyclodextrin of the emulsion according to claim 8 is a pharmaceutically acceptable cyclodextrin.

11. Pharmaceutical formulation according to claim 10 comprising a TGF-β1 inhibitor peptide in an amount of 0.01% to 1% w/w, a β- or a γ-cyclodextrin in an amount of 1% to 10% w/w, cetyl PEG/PPG-10/1 dimethicone in an amount of 0.5% to 6% w/w, a paraben preservative in an amount of 0.005% to 0.6% w/w, a silicone in an amount of 3% to 20% w/w, a mineral oil in an amount of 10% to 40% w/w, and water in q.s.p. 100%.

12. A procedure for the preparation of a complex according to any one of claims 1 to 3, comprising the mixture of the TGF-β1 inhibitor peptide with an aqueous solution comprising the cyclodextrin or a derivative thereof.

13. A procedure for the preparation of a TGF-β1 inhibitor peptide emulsion according to claim 8, comprising
a) the preparation of (i) a complex according to any one of claims 1 to 3; and (ii) a lipophilic phase comprising cetyl PEG/PPG-10/1 dimethicone; and
b) the admixture of said complex onto said lipohilic phase.

14. Procedure according to claim 13, wherein prior to step b), the complex and the lipophilic phase are heated at a temperature comprised between 25°C and 70°C.

15. A product obtainable by a procedure according to any one of claims 12 to 14.

16. Use of a complex according to any one of claims 1 to 3, or a emulsion according to any one of claims 6 to 9, or a pharmaceutical formulation according to any one of claims 4,5, 10 or 11, for the manufacture of a medicament for the treatment of a disease or condition mediated by TGF-β1.

17. Complex according to any one of claims 1 to 3, or emulsion according to any one of claims 6 to 9, or pharmaceutical formulation according to any one of claims 4, 5, 10 or 11, for use in the treatment of a disease or condition mediated by TGF-β1.

18. A method of treating a disease or condition mediated by TGF-β1 in a mammal in need of the treatment, said method comprising the administration to said mammal of an effective amount of a complex according to any one of claims 1 to 3, or an emulsion according to any one of claims 6 to 9, or a pharmaceutical formulation according to any one of claims 4, 5, 10 or 11.
